# EUROPEAN PATENT SPECIFICATION

(11) **EP 3 237 435 B1**
(45) Date of publication and mention of the grant of the patent: **24.05.2023**
(21) Application number: 15817861.6
(22) Date of filing: 23.12.2015
(51) Int. Cl.: C07K 7/08, C07K 14/47

(54) **METABOLICALLY STABLE APELIN ANALOGS IN THE TREATMENT OF DISEASE MEDIATED BY THE APELIN RECEPTOR**
METABOLISCH STABILE APELINANALOGA BEI DER BEHANDLUNG VON DURCH DEN APELINREZEPTOR VERMITTELTEN KRANKHEITEN
ANALOGUES D'APÉLINE MÉTABOLIQUEMENT STABLES DANS LE TRAITEMENT DES MALADIES INDUITES PAR LE RÉCEPTEUR D'APÉLINE

(30) Priority: 23.12.2014 EP 14307170
(43) Date of publication of application: 01.11.2017
(73) Proprietor: INSERM - Institut National de la Santé et de la Recherche Médicale, 75013 Paris (FR); College de France, 75005 Paris (FR); Centre National de la Recherche Scientifique (C.N.R.S.), 75016 Paris (FR); Université de Strasbourg, 67081 Strasbourg cedex (FR)
(72) Inventor: LLORENS-CORTES, Catherine, 75005 Paris (FR); BONNET, Dominique, 67401 Strasbourg (FR); ITURRIOZ, Xavier, 75231 Paris Cedex 05 (FR)
(74) Representative: Inserm Transfert
(86) International application number: PCT/EP2015/081122
(87) International publication number: WO 2016/102648

(56) References cited:
- WO-A1-2014/099984
- WO-A2-2014/081702
- US-A1- 2014 275 489
- ITURRIOZ X ET AL: "[Functional dissociation between apelin receptor signaling and endocytosis: implications for the effects of apelin on arterial blood pressure].", ARCHIVES DES MALADIES DU COEUR ET DES VAISSEAUX AUG 2007, vol. 100, no. 8, August 2007 (2007-08), pages 704-708, XP009184056, ISSN: 0003-9683

## Description

### FIELD OF THE DISCLOSURE:

The disclosure relates to metabolically stable apelin analogs and their use for the prevention or the treatment of disease mediated by the apelin receptor in particular of cardiovascular disease (heart failure, kidney failure, hypertension, pulmonary hypertension) and the syndrome of inappropriate antidiuretic hormone (SIADH).

### BACKGROUND OF THE INVENTION:

Searching for a receptor specific for angiotensin III, the inventors previously isolated from a rat brain cDNA library, a gene encoding a G-protein coupled receptor (GPCR) with seven transmembrane domains (1). The amino-acid sequence of this receptor was 31% identical to that of the rat angiotensin receptor type1( AT1 receptor) and 90% identical to that of the orphan human receptor APJ previously cloned by O'Dowd et al. (2). The endogenous ligand of the human APJ receptor was discovered by Tatemoto et al. (3) and was named apelin. Apelin is a 36-amino acid peptide (apelin 36) generated from a larger precursor of 77 amino acids, preproapelin. The alignment of the preproapelin amino acid sequences in mammalians has demonstrated strict conservation of the C-terminal 17 amino acids, known as apelin-17 or K17F. Several molecular forms of apelin have been identified: *in vivo* apelin 36, K17F and the pyroglutamyl form of apelin 13 (pE13F) (4-8). WO2014/099984 discloses analogues of apelin-13 and analogues of apelin-17 modified by acetyl groups and PEGylated.

Inventors demonstrated that the rat apelin receptor was negatively coupled to adenylate cyclase and internalized under the action of K17F (9). They also showed in the adult rat brain, that the apelin receptor mRNA was expressed in cerebral structures involved in neuroendocrine control, regulation of food intake and body fluid homeostasis (1). They showed the presence of apelinergic neurons in these structures by immunohistochemistry (10). They subsequently showed that apelin and its receptor were co-localized with arginine vasopressin (AVP) in magnocellular vasopressinergic neurons of the paraventricular nucleus (PVN) and the supraoptic nucleus (SON) (4, 11, 12). These neurons project to the posterior pituitary, where they release AVP into the bloodstream. Subsequently, AVP by acting at the kidney level on AVP receptors type 2 (V2 receptors), located in collecting ducts, activates water channels, the aquaporin-2, facilitating their insertion in the apical membrane, resulting in diuresis reduction (antidiuretic effect). They then showed in lactating rats exhibiting hyperactivity of vasopressinergic neurons (making it possible to maintain body water content to optimise milk production) that central injection of K17F decreased the phasic electrical activity of these neurons, resulting in a decrease in the secretion of AVP into the bloodstream and an increase in aqueous diuresis (4). These data suggest that apelin may be a natural inhibitor of the antidiuretic effect of AVP.

In addition to a central action, the aquaretic effect of apelin probably involves a renal action because mRNA transcripts of apelin receptor and preproapelin, as well as apelin peptide, have been detected in rat and human kidney (5, 13). Apelin receptor mRNA has been detected in all renal zones, most abundantly in the inner stripe of the outer medulla (14). A high level of expression was also detected in the glomeruli and a moderate expression was observed in all nephron segments, especially in collecting ducts that express vasopressin V2 receptors. In agreement with this localization, the intravenous (iv) injection, in lactating rats, of apelin in increasing doses, dose-dependently increases diuresis (14). Moreover, inventors have shown (15) that this effect is due to a decrease in the insertion of aquaporin-2 at the apical membrane in the collecting duct. This is due to the inhibitory effect of apelin, on the AVP-induced cAMP production via V2 receptors. Thus, by adjusting the water output to counteract the changes in plasma solute concentration, apelin and AVP could prevent that osmolarity changes more than a few percent of the mean basal level.

Moreover, the dehydration of rats for 24 h, which increases the secretion of AVP into the bloodstream and leads to a decrease in the neuronal AVP content of magnocellular AVP neurons, decreases apelin concentration in parallel plasma and increases the accumulation of apelin content in these neurons. This indicates that, during dehydration, apelin and AVP are regulated in opposite manners, thereby optimizing AVP secretion into the bloodstream and decreasing diuresis to avoid additional water loss at the kidney level (4, 16). They also showed for the first time that plasma apelin levels in humans are regulated by osmotic and volemic stimuli in the opposite direction to AVP suggesting that apelin, like AVP, may participate in the maintenance of body fluid homeostasis not only in rodents but also in humans (17). More recently, inventors observed in a study including hyponatremic patients with the syndrome of inappropriate antidiuretic hormone (SIADH) or with chronic heart failure that an abnormal apelin/AVP balance in plasma might contribute to the water metabolism defect observed in these patients (18).

Apelin is also present in the cardiovascular system. Apelin receptor mRNA has been detected in the myocardium and vascular endothelium (7). Systemic injection of apelin in rats was shown to decrease (blood pressure (BP) (9, 19) via nitric oxyde production (19). Finally, apelin receptor-deficient mice exhibit an increased vasopressor response to angiotensin II, and the base-line BP of double mutant mice homozygous for both the apelin receptor and the AT1 receptor was significantly elevated compared with that of AT1 receptor-deficient mice (20). This demonstrates that apelin exerts a hypotensive effect *in vivo* and plays a counter-regulatory role against the pressor action of angiotensin II. On the other hand, in rodent hearts, apelin increases the contractile force of the myocardium by a positive inotropic effect, while decreasing cardiac loading (21, 22). In addition, an increase in apelin immunoreactivity has been observed in the plasma of patients in the early stages of heart failure, whereas a decrease is observed at later, more severe stages (23). Moreover, apelin receptor mRNA has been shown to be decreased in rat hypertrophied and failing hearts (24). Finally, apelin gene-deficient mice were shown to develop an impaired heart contractility and progressive heart failure associated with aging and pressure overload (25). Therefore, down-regulation of the apelin system seems to coincide with declining cardiac performance raising the possibility that apelin could be a protective agent for cardiac function. Together these data demonstrate that apelin plays a key role in the maintenance of body fluid homeostasis and cardiovascular functions.

Since the half-life of apelin in the blood circulation is around one minute, this invention aims at designing, synthesizing and testing novel potent and stable drugs that activate the apelin/apelin receptor pathway. Such a compound constitutes a potential new therapeutic agent to treat diseases mediated by the apelin receptor in particular cardiovascular diseases (heart failure, kidney failure, hypertension, pulmonary hypertension) and the syndrome of inappropriate antidiuretic hormone (SIADH), especially in heart failure patients by increasing aqueous diuresis and myocardium contractility whilst decreasing vascular resistances

Heart failure constitutes a major and growing health burden in developed countries. In Europe, the European Society of Cardiology (ESC) represents countries with a population of over 900 million, and there are at least 15 million patients with heart failure (26). In the United States, heart failure affects nearly 5,800,000 people (27). Heart failure incidence approaches 10 per 1,000 population after age 65 (28). In the United States, heart failure causes 280,000 deaths annually, and the estimated direct and indirect cost of heart failure for 2010 is $39,2 billion (27). The increasing burden of heart failure in western societies reflects 2major factors: 1) ageing population with higher incidence of heart failure, and 2) more patients surviving an acute myocardial infarction (MI) resulting in development of heart failure. Treatment options depend on the type, cause, symptoms and severity of the heart failure, including treating the underlying causes and lifestyle changes. A number of medications are prescribed for heart failure, and most patients will take more than one drug. Medications may be prescribed to dilate blood vessels (e.g. angiotensin I converting enzyme (ACE) inhibitors or AT1 receptor blockers), strengthen the heart's pumping action (e.g. digoxin) or reduce water and sodium in the body to lessen the heart's workload (e.g. diuretics). However, only ACE inhibitors, AT1 receptor blockers and β-adrenergic receptor blockers have been proofed in large clinical trials to decrease morbidity and mortality in heart failure patients (29, 30, 31 , 32 , 33 , 34, 35). Despite the advancements obtained in medical therapy, the death rate of heart failure remains high: almost 50% of people diagnosed with heart failure will die within 5 years (36, 37). New pharmacological treatments of heart failure are being actively investigated to improve the care of patients. Since the half-life of apelin in the blood circulation is in the minute range, the global aim of this invention is to demonstrate the therapeutic interest of using metabolically stable apelin analogs (apelin receptor agonists) as therapeutic agents useful for treatment of diseases mediated by the apelin receptor in particular of cardiovascular diseases (heart failure, kidney failure, hypertension, pulmonary hypertension), polycystic kidney disease, hyponatremia and SIADH.

### SUMMARY OF THE INVENTION:

The invention provides an apelin analogue having the peptide of the following formula (I):
Lysine-Phenylalanine-Xaa1-Arginine-Xaa2-Arginine-Proline-Arginine-Xaa3-Serine- Xaa4-Lysine-XaaS-Proline-Xaa6- Proline-Xaa7 (I), wherein :
- a fluorocarbon group, an acetyl group, or an acyl group - with the following structure: CH3-CyHx-C(O)-, where y = 7 to 30, x = 2y, is linked to said peptide, directly or through a spacer selected from the group consisting of Lysine and Arginine, either on the alpha-amino or the epsilon-amino group of the first lysine of the peptide of formula (I), and when the spacer is a Lysine, the fluorocarbon group or acetyl group or acyl group is directly linked either on the alpha-amino or the epsilon-amino group of said spacer, and wherein
   Xaa1 is arginine (R) or D-isomer arginine (**R**_{D}).
   Xaa2 is glutamine (Q) or D-isomer glutamine (**Q**_{D})
   Xaa3 is leucine (**L**) or D-isomer Leucine (**L**_{D}).
   Xaa4 is histidine (**H**) or α-aminoisobutyric acid (Aib),
   Xaa5 is alanine (**A**) or D-isomer alanine (**A**_{D}) or glycine (**G**).
   Xaa6 is Methionine (**M**), or Norleucine (Nle).
   Xaa7 is phenylalanine (**F**) or 4-Br phenylalanine (**F**) and
wherein said fluorocarbon group linked to said peptide has the following structure: CmFn-CyHx-(L)-, where m = 3 to 30, n <= 2m + 1, y = 0 to 15, x <= 2y, (m + y) = 3- 30 and (L) which is optional, is a functional group resulting from covalent attachment to the peptides, preferably a carbonyle -C(O)- to form an amide bond to a lysine of said peptide. Said apelin analogue is advantageously metabolically stable.

In preferred embodiments, Xaa1 is D-isomer arginine (**R**_{D}), Xaa2 is D-isomer glutamine (**Q**_{D}), Xaa3 is D-isomer Leucine (**L**_{D}), Xaa4 is α-aminoisobutyric acid (Aib), Xaa5 is D-isomer alanine (**A**_{D}), Xaa6 is Norleucine (Nle), Xaa7 is 4-Br phenylalanine (4BrF).

The invention further relates to an apelin analogue according to the invention for use as a drug.

The invention further relates to a pharmaceutical composition comprising an apelin analogue of the invention and one or more pharmaceutically acceptable excipient.

The invention further relates to a metabolically stable apelin analogue of the invention or a pharmaceutical composition of the invention for use in the treatment or in the prevention of a disease, condition or disorder mediated by the apelin in a mammals in need thereof.

In particular, the present invention is defined by the claims.

The following figures and example do not fall under the scope of the present disclosure and are present for understanding and illustration purposes only.

### DETAILED DESCRIPTION OF THE INVENTION:

Since apelin is rapidly metabolized (half-life of K17F in the blood circulation: (40 seconds, personal data), metabolically stable apelin analogs activating the apelin/apelin receptor pathway are required to determine the therapeutic potential of increasing apelin signalling in patients with heart failure. With this aim, the inventors performed structure-activity relation studies of apelin 13 (pE13F) and apelin 17 (K17F: SEQ ID NO:1 : KFRRQRPRLSHKGPMPF). The inventors obtained metabolically stable apelin analogs (P92 and *JFM V-0196B* compounds), the most potent of which was compound P92. This compound displayed towards rat apelin receptor a Ki of 0.2 ± 0.06 nM determined by competitive radioligand binding assay with [125I] pE13F. Its selectivity towards the AT1 receptor is of a factor 100 with respect to the apelin receptor. This compound behaves as a full agonist on inhibition of cAMP production induced by forskolin and towards apelin receptor internalization. Intracerebroventricular (i.c.v.) injection of increasing doses of P92 in water-deprived mice induced a dose-dependent decrease in plasma AVP levels with a higher potency than that induced by i.c.v. injection of K17F. Moreover, P92 induced a vasorelaxation of aortic rings pre-constricted with noradrenaline or glomerular arterioles precontracted by angiotensin II similar to that induced by K17F. Injection by the intravenous route of P92 in anaesthesized normotensive rats in increasing doses, dose-dependently decreases dose-dependently arterial blood pressure (BP). P92 applied on isolated perfused rat heart increases cardiac contractility. Furthermore, the compound of the invention (P92 and *JFM V-0196B* compounds) shows increase half-life stability in plasma.

The inventors have thus discovered a new targeted therapy for treating diseases mediated by the apelin receptor. Said invention is particularly advantageous for treating cardiovascular diseases (heart failure, hypertension) and the syndrome of inappropriate antidiuretic hormone (SIADH).

### Definitions:

Throughout the specification, several terms are employed and are defined in the following paragraphs.

As used herein, the term "Apelin" is the endogenous ligand of the apelin receptor and is synthesized as a 77-amino acid prepropeptide processed into C-terminal fragments denoted as Apelin-36, Apelin-17 (K17F) and the pyroglumyl form of Apelin-13 (pE13F).

Apelin is expressed in endocardial and vascular endothelial cells while the apelin receptor is widely distributed, allowing for autocrine and paracrine cardiovascular effects. Apelin mediates a positive inotropic effect and centrally inhibits vasopressin release and promotes diuresis. The apelin/apelin receptor axis is pro-angiogenic and activates endothelial NO leading to vasodilatation. Loss of apelin exacerbates post-MI (Myocardial Infarcts) dysfunction, Heart failure (HF) and Pulmonary arterial hypertension (PAH). Apelin peptides stimulate vascular and cardiac stem cells thereby facilitating tissue injury reparative actions. Genetic variation in apelin receptor modifies the progression of HF in dilated cardiomyopathy and the apelin/ apelin receptor system is compromised in human HF and PAH. Apelin administration increased cardiac index and lowered peripheral vascular resistance in the absence of hypotension in patients with HF. PAH is associated with marked inflammation and vascular remodeling and is a stereotypical example of a vascular disease with limited therapy. Apelin peptides play a key role in inflammatory vascular diseases in pathological states including PAH. However, current therapeutic applications are not feasible due to the short half-life (1-2 mins) of native apelin peptides thereby compromising its commercial applicability.

The term "APJ receptor" or "apelin receptor" means the receptor for apelin originally identified by O'Dowd et al from a human genomic library (2) and subsequently cloned in mice (38) and rats ((1), our laboratory).

As used herein, the term "amino acid" refers to natural or unnatural amino acids in their D and L stereoisomers for chiral amino acids. It is understood to refer to both amino acids and the corresponding amino acid residues, such as are present, for example, in peptidyl structure. Natural and unnatural amino acids are well known in the art. Common natural amino acids include, without limitation, alanine (Ala), arginine (Arg), asparagine (Asn), aspartic acid (Asp), cysteine (Cys), glutamine (Gln), glutamic acid (Glu), glycine (Gly), histidine (His), isoleucine (Ile), leucine (Leu), lysine (Lys), methionine (Met), phenylalanine (Phe), proline (Pro), serine (Ser), threonine (Thr), tryptophan (Trp), tyrosine (Tyr), and valine (Val). Uncommon and unnatural amino acids include, without limitation, α-aminoisobutyric acid (Aib), allyl glycine (AllylGly), norleucine (Nle), norvaline, biphenylalanine (Bip), citrulline (Cit), 4-guanidinophenylalanine (Phe(Gu)), homoarginine (hArg), homolysine (hLys), 2-naphtylalanine (2-Nal), ornithine (Om) and pentafluorophenylalanine.

Amino acids are typically classified in one or more categories, including polar, hydrophobic, acidic, basic and aromatic, according to their side chains. Examples of polar amino acids include those having side chain functional groups such as hydroxyl, sulfhydryl, and amide, as well as the acidic and basic amino acids. Polar amino acids include, without limitation, asparagine, cysteine, glutamine, histidine, selenocysteine, serine, threonine, tryptophan and tyrosine. Examples of hydrophobic or non-polar amino acids include those residues having nonpolar aliphatic side chains, such as, without limitation, leucine, isoleucine, valine, glycine, alanine, proline, methionine and phenylalanine. Examples of basic amino acid residues include those having a basic side chain, such as an amino or guanidino group. Basic amino acid residues include, without limitation, arginine, homolysine and lysine. Examples of acidic amino acid residues include those having an acidic side chain functional group, such as a carboxyl group. Acidic amino acid residues include, without limitation aspartic acid and glutamic acid. Aromatic amino acids include those having an aromatic side chain group. Examples of aromatic amino acids include, without limitation, biphenylalanine, histidine, 2-napthylalananine, pentafluorophenylalanine, phenylalanine, tryptophan and tyrosine. It is noted that some amino acids are classified in more than one group, for example, histidine, tryptophan and tyrosine are classified as both polar and aromatic amino acids. Amino acids may further be classified as non-charged, or charged (positively or negatively) amino acids. Examples of positively charged amino acids include without limitation lysine, arginine and histidine. Examples of negatively charged amino acids include without limitation glutamic acid and aspartic acid. Additional amino acids that are classified in each of the above groups are known to those of ordinary skill in the art.

"Equivalent amino acid" means an amino acid which may be substituted for another amino acid in the peptide compounds according to the invention without any appreciable loss of function. Equivalent amino acids will be recognized by those of ordinary skill in the art. Substitution of like amino acids is made on the basis of relative similarity of side chain substituents, for example regarding size, charge, hydrophilicity and hydrophobicity as described herein. The phrase "or an equivalent amino acid thereof" when used following a list of individual amino acids means an equivalent of one or more of the individual amino acids included in the list.

As used herein, an "apelin analogue" refers to a compound exhibiting at least one, preferably all, of the biological activities of a peptide of SEQ ID NO: 1. The apelin analogue may for example be characterized in that it is capable of activating the apelin/apelin receptor pathway through experiments (see Example).

As used herein a "metabolically stable" apelin analogue refers to an apelin analogue which has a half-life superior to K17F (see test described in Example for P92 and *JFM V-0196B* compounds). Preferably, a "metabolically stable" apelin analogue refers to an apelin analogue which has a half-life at twice longer than K17F half-life, or at least 20 min or more than one hour, as measured in the test described in the Example for P92 and *JFM V-0196B* compounds.

A peptide "substantially homologous" to a reference peptide may derive from the reference sequence by one or more conservative substitutions. Preferably, these homologous peptides do not include two cysteine residues, so that cyclization is prevented. Two amino acid sequences are "substantially homologous" or "substantially similar" when one or more amino acid residue are replaced by a biologically similar residue or when greater than 80 % of the amino acids are identical, or greater than about 90 %, preferably greater than about 95%, are similar (functionally identical). Preferably, the similar, identical or homologous sequences are identified by alignment using, for example, the GCG (Genetics Computer Group, Program Manual for the GCG Package, Version 7, Madison, Wisconsin) pileup program, or any of the programs known in the art (BLAST, FASTA, etc.). The percentage of identity may be calculated by performing a pairwise global alignment based on the Needleman-Wunsch alignment algorithm to find the optimum alignment (including gaps) of two sequences along their entire length, for instance using Needle, and using the BLOSUM62 matrix with a gap opening penalty of 10 and a gap extension penalty of 0.5.

The term "conservative substitution" as used herein denotes the replacement of an amino acid residue by another, without altering the overall conformation and function of the peptide, including, but not limited to, replacement of an amino acid with one having similar properties (such as, for example, polarity, hydrogen bonding potential, acidic, basic, shape, hydrophobic, aromatic, and the like). Amino acids with similar properties are well known in the art. For example, arginine, histidine and lysine are hydrophilic-basic amino acids and may be interchangeable. Similarly, isoleucine, a hydrophobic amino acid, may be replaced with leucine, methionine or valine. Neutral hydrophilic amino acids, which can be substituted for one another, include asparagine, glutamine, serine and threonine.

By "substituted" or "modified" the present invention includes those amino acids that have been altered or modified from naturally occurring amino acids.

As used herein, the term "pharmaceutically acceptable" and grammatical variations thereof, as they refer to compositions, carriers, diluents and reagents, are used interchangeably and represent that the materials are capable of administration to or upon a mammal without the production of undesirable physiological effects such as nausea, dizziness, gastric upset and the like.

The term "patient" or "subject" refers to a human or non human mammal, preferably a mouse, cat, dog, monkey, horse, cattle (i.e. cow, sheep, goat, buffalo), including male, female, adults and children.

As used herein, the term "treatment" or "therapy" includes curative and/or prophylactic treatment. More particularly, curative treatment refers to any of the alleviation, amelioration and/or elimination, reduction and/or stabilization (e.g., failure to progress to more advanced stages) of a symptom, as well as delay in progression of a symptom of a particular disorder. Prophylactic treatment refers to any of: halting the onset, reducing the risk of development, reducing the incidence, delaying the onset, reducing the development, as well as increasing the time to onset of symptoms of a particular disorder.

### APELIN ANALOGS

The disclosure relates to novel apelin analogs derived from the apelin K17F isoform, which have the ability to activate the apelin/apelin receptor pathway; and/or to treat diseases mediated by the apelin receptor, in particular cardiovascular diseases (heart failure, renal failure, hypertension), polycystic kidney disease, hyponatremia and inappropriate vasopressin secretions (SIADH).

In one aspect, the invention provides an apelin analogue having the peptide of the following formula (I):
Lysine-Phenylalanine-Xaa1-Arginine-Xaa2-Arginine-Proline-Arginine-Xaa3-Serine- Xaa4-Lysine-XaaS-Proline-Xaa6- Proline-Xaa7 (I), wherein :
- a fluorocarbon group, an acetyl group, or an acyl group - with the following structure: CH3-CyHx-C(O)-, where y = 7 to 30, x = 2y,is linked to said peptide, directly or through a spacer selected from the group consisting of Lysine and Arginine, either on the alpha-amino or the epsilon-amino group of the first lysine of the peptide of formula (I), and when the spacer is a Lysine, the fluorocarbon group or acetyl group or acyl group is directly linked either on the alpha-amino or the epsilon-amino group of said spacer, and wherein
   Xaa1 is arginine (**R**) or D-isomer arginine (**R**_{D}).
   Xaa2 is glutamine (**Q**) or D-isomer glutamine (**Q**_{D})
   Xaa3 is leucine (**L**) or D-isomer Leucine (**L**_{D}).
   Xaa4 is histidine (**H**) or α-aminoisobutyric acid (Aib),
   Xaa5 is alanine (**A**) or D-isomer alanine (**A**_{D}) or glycine.
   Xaa6 is Methionine (**M**), or Norleucine (Nle).
   Xaa7 is phenylalanine (**F**) or 4-Br phenylalanine (**F**) and
   wherein said fluorocarbon group linked to said peptide has the following structure: CmFn-CyHx-(L)-, where m = 3 to 30, n <= 2m + 1, y = 0 to 15, x <= 2y, (m + y) = 3- 30 and (L) which is optional, is a functional group resulting from covalent attachment to the peptides, preferably a carbonyle -C(O)- to form an amide bond to a lysine of said peptide..

Said apelin analogue is advantageously metabolically stable.

As used herein, the term « fluorocarbon » includes either, perfluorocarbon (where all hydrogen are replaced by fluor) or, hydrofluorocarbon (which contains both C-H and C-F bonds).

The fluorocarbon group may comprise one or more chains derived from perfluorocarbon or mixed fluorocarbon/hydrocarbon radicals, and may be saturated or unsaturated, each chain having from 3 to 30 carbon atoms. The fluorocarbon group is linked to the peptide through a covalent linkage, for example via NH2-, group of a Lysine of the peptide of formula I. The coupling to the peptide may be achieved through functional group for linkage to -NH₂, being naturally present on the Lysine of the peptide of formula I, or onto a spacer. Examples of such linkages include amide, hydrazine, disulphide, thiother and oxime bonds.

Optionally, a cleavable spacer element (peptidic or non-peptidic) may be incorporated to permit cleavage of the peptide from the fluorocarbon group. The spacer may also be incorporated to assist in the synthesis of the molecule and to improve its stability and/or solubility. Examples of spacers include amino acids such as lysine or arginine that may be cleaved by proteolytic enzymes.

Thus, the fluorocarbon group of the apelin analogue according to the present invention has chemical structure CmFn-CyHx-(L)-, where m = 3 to 30, n <= 2m + 1, y = 0 to 15, x <= 2y, (m + y) = 3- 30 and (L) which is optional, is a functional group resulting from covalent attachment to the peptides. For example said functional group is a carbonyl group forming an amide bond with the -NH2 of a lysine. In further related specific embodiments, m= 5 to 15. In other specific embodiments, m= 5 to 15 and y = 1 to 4.

In a particular embodiment of the above formula the fluorocarbon group results from the linkage of perfluoroundecanoid acid of the formula A. 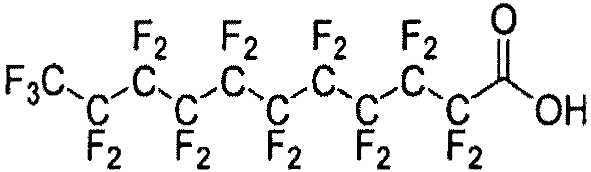 or alternatively 2H, 2H, 2H, 3H, 3H-perfluoroundecanoid acid of the formula (B)

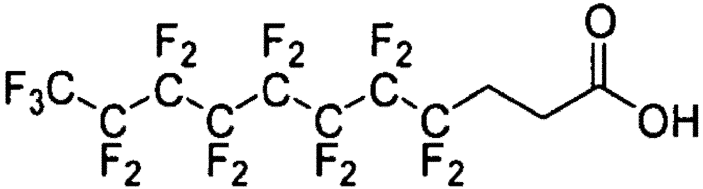

Or heptadecafluoro-pentadecanoic acid of the formula (C)

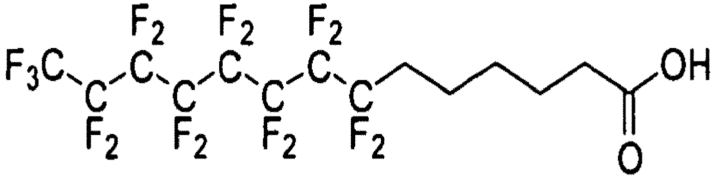

In other specific embodiments, said acyl group of the apelin analogue according to the present invention has the following structure:
CH3-CyHx-C(O)-, where y = 7 to 30, x = 2y. In further related specific embodiments, y= 10 to 20. For example, y=14.

The fluorocarbon group or RC(O)- acyl group could be linked at the N-terminal part of the peptide directly through a lysine, either on the alpha-amino or the epsilon-amino groups.

In some embodiments, Xaa1 is D-isomer arginine (**R**_{D}), Xaa2 is D-isomer glutamine (**Q**_{D}), Xaa3 is D-isomer Leucine (**L**_{D}), Xaa4 is α-aminoisobutyric acid (Aib), Xaa5 is D-isomer alanine (**A**_{D}), Xaa6 is Norleucine (Nle), Xaa7 is 4-Br phenylalanine (4-Br F).

In particular embodiment, the invention provides an apelin analogue selected from the group consisting of:
i) Acetyl-Lys-Phe-(D-Arg)-Arg-(D-Gln)-Arg-Pro-Arg-(D-Leu)-Ser-Aib-Lys-(D-Ala)-Pro-Nle-Pro-(4-Br)Phe (P92 compound);
ii) A peptide of the amino acid sequence of SEQ ID NO:1 (KFRRQRPRLSHKGPMPF) with a fluorocarbon group linked at the NH2α terminal (*JFM V-0196B* compound)
iii) A peptide of the amino acid sequence of SEQ ID NO:1 (KFRRQRPRLSHKGPMPF) with a fluorocarbon group linked at the NH2ε of the first lysine residue (*JFM V-0220B* compound)
iv) A peptide of the amino acid sequence of SEQ ID NO:1 (KFRRQRPRLSHKGPMPF) with a fluorocarbon group linked at the εNH2 of the lysine residue of the linker L Lysine (*JFM V-0210*/*1* compound)
v) A peptide of the amino acid sequence of SEQ ID NO:2 (KF**R**_{D}R**Q_{D}**RPR**L_{D}**S**Aib**K**A_{D}**P**Nle**P(**4-Br**)**F**) with a fluorocarbon group linked at the NH2α of the first lysine residue (FLUORO-P92 compound)
vi) A peptide of the amino acid sequence of SEQ ID NO:2 (KF**R_{D}**R**Q_{D}**RPR**L_{D}**S**Aib**K**A_{D}**P**Nle**P(**4-Br**)**F**) with an acyl group linked at the NH2α of the first lysine residue (LIPO-P92 compound).

In a preferred embodiment, the apelin analogue comprises or consists of the (i) Acetyl-Lys-Phe-(D-Arg)-Arg-(D-Gln)-Arg-Pro-Arg-(D-Leu)-Ser-Aib-Lys-(D-Ala)-Pro-Nle-Pro-(4-Br)Phe (herein referred as the P92 compound) or (ii) the amino acid sequence of SEQ ID NO:1 (KFRRQRPRLSHKGPMPF) with a fluorocarbon group at the NH2 terminal (herein referred as the *JFM V-0196B* compound) or (iii) KF**R**_{D}R**Q_{D}**RPR**L_{D}**S**Aib**K**A_{D}**PN**le**P(**4-Br**)**F**) with a fluorocarbon group linked at the NH2α of the first lysine residue (FLUORO-P92 compound) or (iv) KF**R**_{D}R**Q_{D}**RPR**L_{D}**S**Aib**K**A_{D}**PN**le**P(**4-Br**)**F**) with an acyl group linked at the NH2α of the first lysine residue (LIPO-P92 compound).

In particular embodiment, the invention provides an apelin analogue selected from the group consisting of:
i) A peptide of the amino acid sequence of SEQ ID NO:1 (KFRRQRPRLSHKGPMPF) with an acyl group RC(O)-,linked at the NH2α terminal (*JFM V-0196A* compound)
ii) A peptide of the amino acid sequence of SEQ ID NO:1 (KFRRQRPRLSHKGPMPF) with an acyl group RC(O)-,linked at the NH2ε of the first lysine residue (*JFM V-0220A* compound)
iii) A peptide of the amino acid sequence of SEQ ID NO:1 (KFRRQRPRLSHKGPMPF) with a acyl group RC(O)-,linked at the εNH2 of the lysine residue of the linker L Lysine (*JFM V-0210*/*2* compound)

Preferably, the apelin analogue according to the invention has the capacity (i) to activate the apelin/apelin receptor pathway and/or to be a metabolically stable apelin receptor agonist.

The person skilled in the art can easily determine whether the apelin analogue is biologically active. For example, the capacity to activate the apelin/apelin receptor pathway can be determined by assessing inhibition of cAMP production induced by forskolin, ERK phosphorylation and towards apelin receptor internalization (e.g. as described in Example). Agonistic activities of an apelin analogue toward APJ receptor may be determined by any well-known method in the art. For example, since the compound of the present invention can promote the function of the apelin receptor, the agonist can be screened by using the natural agonist of APJ receptor (i.e. apelin) and its receptor in a competitive binding test and test associated with the biological activity (see below).

Furthermore, a method for determining whether an apelin analogue is an apelin receptor agonist is described in Iturrioz . et al. (39). The US Patent Application Publication N°US 2005/0112701 also described test system for the identification of a ligand for angiotension receptor like-1 (APJ receptor) comprising an APJ receptor. Another method is also described in the US Patent Publication US 6,492,324.

As such, it should be understood that in the context of the present disclosure, a conservative substitution is recognized in the art as a substitution of one amino acid for another amino acid that has similar properties.

According to the disclosure a first amino acid sequence having at least 80% of identity with a second amino acid sequence means that the first sequence has 80; 81; 82; 83; 84; 85; 86; 87; 88; 89; 90; 91; 92; 93; 94; 95; 96; 97; 98; or 99% of identity with the second amino acid sequence. Amino acid sequence identity is preferably determined using a suitable sequence alignment algorithm and default parameters, such as BLAST P (40).

The synthesis of metabolically stable apelin analogue is described in the Example (Material and Method).

### Pharmaceutical compositions

Another aspect of the present invention includes pharmaceutical compositions prepared for administration to a subject and which include a therapeutically effective amount of one or more of the metabolically stable apelin analogs of the invention, as described above. The therapeutically effective amount of a metabolically stable apelin analogue will depend on the route of administration, the type of mammal that is the subject and the physical characteristics of the subject being treated. Specific factors that can be taken into account include disease severity and stage, weight, diet and concurrent medication. The relationship of these factors to determining a therapeutically effective amount of the disclosed compounds is understood by those of ordinary skill in the art.

More particularly, the invention relates to a pharmaceutical composition comprising a metabolically stable apelin analogue of the invention and one or more pharmaceutically acceptable carrier.

The compound is formulated in association with a pharmaceutically acceptable carrier.

Any of the metabolically stable apelin analogs described herein may be combined with a pharmaceutically acceptable vehicle or excipient to form a pharmaceutical composition. Pharmaceutical vehicles or excipients are known to those skilled in the art. These most typically would be standard vehicles or excipients for administration of compositions to humans, including solutions such as sterile water, saline, and buffered solutions at physiological pH. The compositions can also be administered intramuscularly, subcutaneously, or in an aerosol form. Other compounds are administered according to standard procedures used by those skilled in the art. Pharmaceutical excipients include thickeners, diluents, buffers, preservatives, surface active agents and the like in addition to the molecule of choice. Descriptions of some of these pharmaceutically acceptable excipients or vehicles may be found in The Handbook of Pharmaceutical Excipients, published by the American Pharmaceutical Association and the Pharmaceutical Society of Great Britain. Remington: the Science and Practice of Pharmacy 20th edition (2000), describes compositions and formulations suitable for pharmaceutical delivery of the compounds of the invention, in the form of aqueous solutions, lyophilized or other dried formulations. Pharmaceutical compositions can also include one or more additional active ingredients such as anti-hypertensive agents, anti-inflammatory agents, and the like.

In general, the nature of the vehicle will depend on the particular mode of administration being employed. For instance, parenteral formulations usually contain injectable fluids that include pharmaceutically and physiologically acceptable fluids such as water, physiological saline, balanced salt solutions, aqueous dextrose, glycerol or the like as a vehicle.

In solid oral preparations, for example, powders, granules, capsules, caplets, gelcaps, pills and tablets (each including immediate release, timed release and sustained release formulations), suitable vehicles and excipients include but are not limited to diluents, granulating agents, lubricants, binders, glidants, disintegrating agents and the like. Because of their ease of administration, tablets and capsules represent the most advantageous oral dosage unit form, in which solid pharmaceutical excipients are obviously employed. If desired, tablets may be sugar coated, gelatin coated, film coated or enteric coated by standard techniques.

Preferably these compositions are in unit dosage forms, such as tablets, pills, capsules, powders, granules, lozenges, sterile parenteral solutions or suspensions, metered aerosol or liquid sprays, drops, ampoule, autoinjector devices, or suppositories for administration by oral, intranasal, sublingual, intraocular, transdermal, parenteral, rectal, vaginal or insufflation means.

In addition to biologically-neutral carriers, pharmaceutical compositions to be administered can contain minor amounts of non-toxic auxiliary substances, such as wetting or emulsifying agents, preservatives, and pH buffering agents and the like, for example sodium acetate or sorbitan monolaurate.

In a specific embodiment, the compositions are formulated for their administration into the airways, e.g. by inhalation. The pharmaceutical composition of the invention may thus be formulated as solution appropriate for inhalation.

The dosing is selected by the skilled person so that an anti-infectious effect is achieved, and depends on the route of administration and the dosage form that is used. Total daily dose of a peptide administered to a subject in single or divided doses may be in amounts, for example, of from about 0.001 to about 100 mg/kg body weight daily and preferably 0.01 to 10 mg/kg/day. Dosage unit compositions may contain such amounts of such submultiples thereof as may be used to make up the daily dose. It will be understood, however, that the specific dose level for any particular patient will depend upon a variety of factors including the body weight, general health, sex, diet, time and route of administration, rates of absorption and excretion, combination with other drugs and the severity of the particular disease being treated.

### Therapeutic applications

The metabolically stable apelin analogue of the invention, the pharmaceutical composition of the invention is used for treating diseases mediated by the apelin receptor in particular cardiovascular diseases (heart failure, kidney failure, hypertension, pulmonary hypertension), polycystic kidney disease, hyponatremia and SIADH.

In particular, the metabolically stable apelin analogue of the invention has the ability to decrease the hypertension in a subject of at least 50%, 60%, 70%, 80%, 90% or 100%.

The invention also provides a method of treatment of a disease mediated by the apelin receptor in a patient in need thereof, which method comprises administering said patient with a metabolically stable apelin analogue of the invention.

The role of apelin in the pathophysiology of various diseases has been described in (41).

Accordingly, the metabolically stable apelin analogue of the invention is suitable for the modulation of the central nervous system function (vasopressin neuron activity and systemic vasopressin release, drinking behavior, food intake), the cardiovascular function (blood pressure, myocardium contractibility), the immune function, the gastrointestinal function, the metabolic function, the reproductive function, etc. and therefore, can be used as a therapeutic and/or prophylactic agent for a variety of diseases.

The present invention relates thus to a method for treating and/or preventing a disease, condition or disorder mediated by the apelin in a mammals in need thereof, such method involving the step of administering to a mammal in need thereof a therapeutically effective amount of a metabolically stable apelin analogue of the present invention or a pharmaceutical composition thereof.

Diseases, conditions and/or disorders which can be treated or prevented by the administration of a metabolically stable apelin analogue are for example:
- cardiovascular diseases: Heart failure, kidney diseases (e.g. renal failure, nephritis, etc.) hypertension, pulmonary hypertension, cirrhosis, arteriosclerosis, pulmonary emphysema, pulmonary oedema; stroke, brain ischemia, myocardial impairment in sepsis
- the syndrome of inappropriate antidiuretic hormone (SIADH) including pathologies like neurogenic diabetes mellitus (e.g. diabetic complications such as diabetic nephropathy, diabetic neuropathy, diabetic retinopathy, etc.), septic choc, thirst troubles;
- metabolic diseases: Obesity, anorexia, hyperphagia, polyphagia, hypercholesterolemia, hyperglyceridemia, hyperlipemia;
- various types of dementia such as senile dementia, cerebrovascular dementia, dementia due to genealogical denaturation degenerative diseases (e.g. Alzheimer's disease, Parkinson's disease, Pick's disease, Huntington's disease, etc.), dementia resulting from infectious diseases (e.g. delayed virus infections such as Creutzfeldt-Jakob disease), dementia associated with endocrine diseases, metabolic diseases, or poisoning (e.g. hypothyroidism, vitamin B12 deficiency, alcoholism, poisoning caused by various drugs, metals, or organic compounds), dementia caused by tumors (e.g. brain tumor), and dementia due to traumatic diseases (e.g. chronic subdural hematoma), depression, hyperactive child syndrome (microencephalopathy), disturbance of consciousness, anxiety disorder, schizophrenia, phobia;
- sarcopenia: a syndrome characterised by progressive and generalised loss of skeletal muscle mass and strength with a risk of adverse outcomes such as physical disability, poor quality of life and death.
- polycystic kidney disease (PKD or PCKD, also known as polycystic kidney syndrome) is a cystic genetic disorder of the kidneys. There are two types of PKD: autosomal dominant polycystic kidney disease (ADPKD) and the less- common autosomal recessive polycystic kidney disease (ARPKD). PKD is caused by loss-of-function mutations in either PKD1 or PKD2;
- hyponatremia is defined as a serum sodium level of less than 135 mEq/L and is considered severe when the serum level is below 125 mEq/L. Many medical illnesses, such as congestive heart failure, liver failure, renal failure, SIADH or pneumonia, may be associated with hyponatremia.

The metabolically stable apelin analogue is be used as a postoperative nutritional status improving agent or as an inotropic agent, vasodilatator or an aqueous diuretic.

In preferred embodiment, the subject suffers from cardiovascular diseases and/or SIADH.

Furthermore, the present disclosure relates to a metabolically stable apelin analogue for use in an anti-aggregant platelet treatment in a subject in need thereof.

As used herein the term "subject" refers to any subject (preferably human). Preferably the subject is afflicted with an ischemic condition or is at risk of having an ischemic condition.

The term "ischemic conditions" refers to any conditions that result from a restriction in blood supply in at least one organ or tissue due to a clot formed by platelet aggregation. These conditions typically result from the obstruction of a blood vessel by a clot. For example ischemic conditions include but are not limited to renal ischemia, retinal ischemia, brain ischemia, leg ischemia and myocardial ischemia.

Apelin analogue of the present disclosure are particularly suitable for preventing the formation of thrombus, which can be either a non-occlusive thrombus or an occlusive thrombus. Particularly, metabolically stable apelin analogue are envisaged to prevent arterial thrombus formation, such as acute coronary occlusion. The metabolically stable apelin analogue of the disclosure are further provided in a method of antithrombotic treatment to maintain the patency of diseased arteries, to prevent restenosis, such as after PCTA or stenting, to prevent thrombus formation in stenosed arteries, to prevent hyperplasia after angioplasty, atherectomy or arterial stenting, to prevent unstable angina, and generally to prevent or treat the occlusive syndrome in a vascular system.

Apelin analogue of the invention may be thus useful for the prevention of thrombosis, and particular venous and arterial thrombosis

Apelin analogue of the invention may also be used to treat patients with acute coronary syndrome, in particular by preventing further events in the coronary arteries.

Apelin analogue of the invention may finally be used to prevent restenosis after vascular injury.

Apelin analogue of the invention may finally be used to treat patients suffering from hyponatremia.

Apelin analogue of the invention may finally be used to treat patients suffering from polycystic kidney disease.

The invention will be further illustrated by the following figures and examples. However, these examples and figures should not be interpreted in any way as limiting the scope of the present invention.

### FIGURES:

**Figure 1****: Effects of K17F, pE13F, P26 and P92 on the internalization of the rat apelin receptor-EGFP stably expressed in CHO cells.**
**Figure 2****: Vasorelaxant effects of K17F, pE13F, P26 and P92.**
   ***2A: on rat aortic rings precontracted by noradrenaline (NA)***
   ***2B: on rat glomerular arterioles precontracted by Angiotensin II (AngII)***
**Figure 3****: Kinetics of the hypotensive effects of K17F and P92 at two doses (3A and 3B) on arterial blood pressure in anaesthesized normotensive rats after intravenous injection.**
**Figure 4****: Dose-response curve to K17F or P92 on Mean Arterial Blood Pressure (MABP) in anaesthesized normotensive rats after intravenous injection.**
**Figure 5****: Kinetics of the hypotensive effects of JFM V-196B on arterial blood pressure after intravenous injection in anaesthetized normotensive rats at three doses**
**Figure 6****: Effects of the intracerebroventricular injection of K17F or P92 on systemic AVP release in alert euhydrated and dehydrated mice.**
**Figure 7****: Effects of K17F and P92 on cardiac contractility in isolated perfused rat heart preparation**

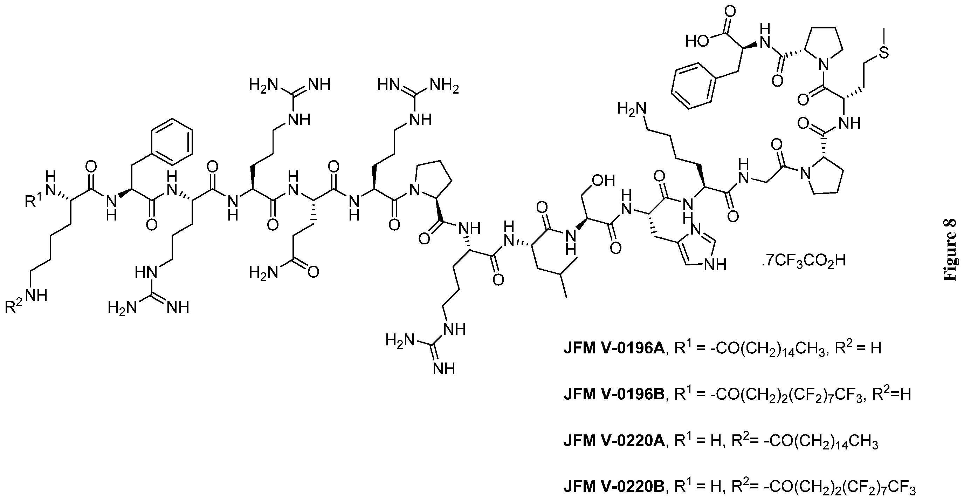

**Figure 8****. Chemical structures of alkyl- and fluorocarbon group peptides**
**Figure 9****: Effects of pE13F, P26, K17F, P92 and JFM V-0196B on rat ApelinR-EGFP internalization in CHO cells.** The ability of pE13F, P26, K17F, P92, K17F and JFM V-0196B to induce the ApelinR internalization was studied in CHO cells stably expressing the rat ApelinR-EGFP treated with increasing concentrations of the different compounds (from 100 pM to 10 nM) for 20 min at 37°C. Cells were then fixed and analyzed by confocal microscopy. Images are representative of the data from at least 3 independent experiments.

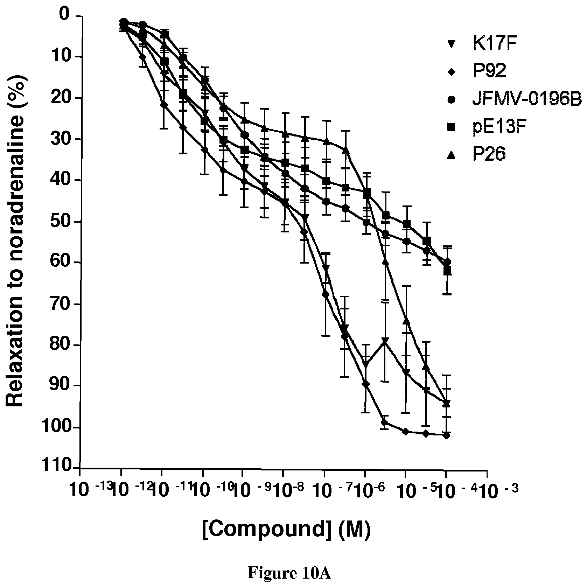

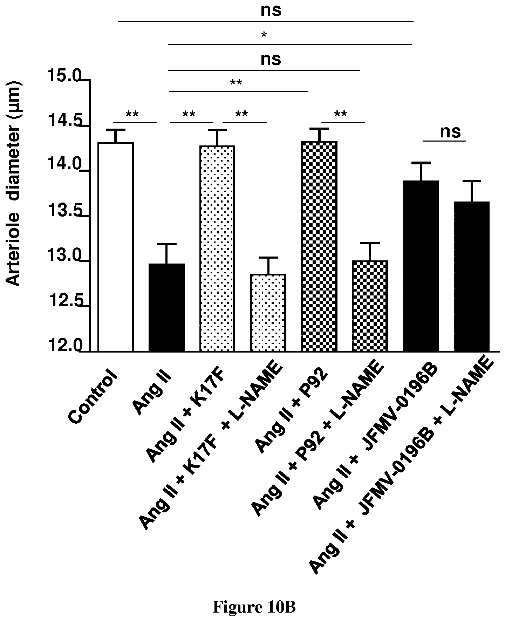

**Figure 10****: Vasorelaxing effects of K17F, pE13F and apelin analogs.** (A) Cumulative concentration-response curves of pE13F (black), P26 (green), K17F (blue), P92 (red) and JFM V-0196B (purple) in rat aorta precontracted by NA (3 µM). (B) Effects of K17F, P92 and JFM V-0196B on the rat glomerular arteriole contractile response to Ang II in the absence or presence of L-NAME (20 µM). Arteriolar diameters were measured in the basal conditions (Control), then 1 min after adding Ang II (10 nM) and 1 min after addition of 500 nM K17F, P92 or JFMV-0196B on AngII-induced vasoconstricted arterioles. Data are means ± SEM of 5-8 independent experiments. ^{∗} *p* < 0.05, ^{∗∗}*p* < 0.01

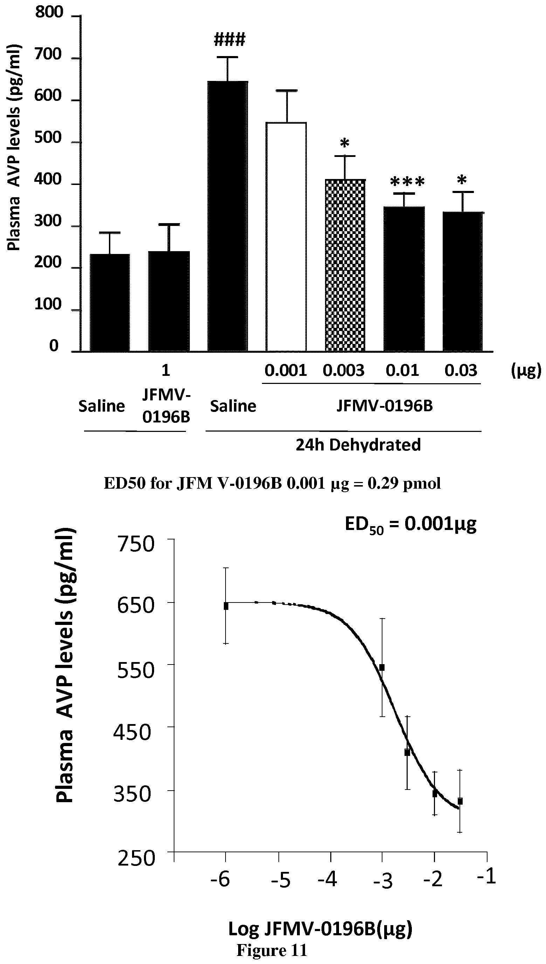

**Figure 11****:** Effects of i.c.v. injection of K17F, P92 and JFM V-0196B in mice on water deprivation-induced systemic AVP release. After 24 h of water deprivation, mice received 10 µl i.c.v. saline or increasing amounts of JFMV-0196B (0.001 to 0.03 µg) or K17F (1µg) and were compared with mice with free access to water that received 10 µl i.c.v. saline or JFM V-0196B (1µg). Plasma AVP levels were determined 1 min after injection by RIA. Histograms represent mean ± SEM of plasma AVP levels (pg/ml) from 7 to 20 animals for each set of conditions. Data were analyzed with GraphPad Prism (GraphPad Software, La Jolla, CA, USA). Statistical comparisons were performed with one-way analysis of variance (ANOVA) followed by Bonferroni's post-test. ### P < 0.001 vs. euhydrated; * P <0.05, *** P < 0.001 vs. water-deprived mice given saline. Insets: sigmoidal curves of JFMV-0196B dose responses on AVP release in conscious water-deprived mice.
**Figure 12****: Chemical structures of FLUORO-P92 compound**
**Figure 13****: Chemical structures of LIPO-P92 compound**

### EXAMPLE: 1 Material & Methods

### _I - Drugs and Radioligand

1) K17F, pE13F and their derivatives were synthesized by PolyPeptide Laboratories (Strasbourg, France) and GL Biochem (Shangaï, China) respectively. ¹²⁵I-pE13F (iodinated on Lysine⁸ by Bolton Hunter) was purchased from Perkin Elmer (Wellesley, MA, USA).
2) The synthesis of alkyl and perfluoroalkylpeptides derived from apelin-13 was performed in the laboratory of Therapeutic Innovation directed by Pr M. Hibert, by Drs D. Bonnet and JF Margathe.

### Scheme 1. Synthesis of alkyl- and perfluoroalkylpeptides

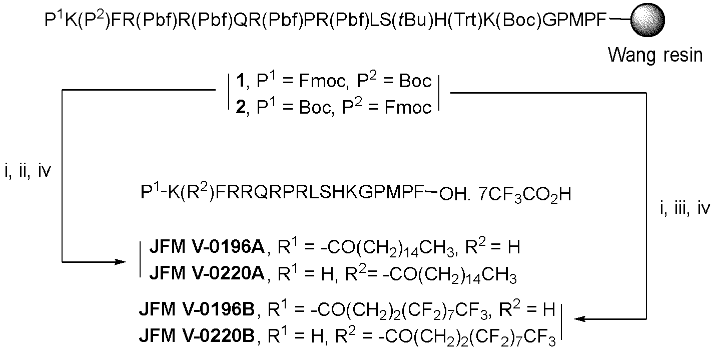

(i) Piperidine/DMF; (ii) CH₃(CH₂)₁₄CO₂H, HBTU, HOBt, DIPEA, DMF; (iii) CF₃(CF₂)₇(CH₂)₂CO₂H, HBTU, HOBt, DIPEA, DMF; (iv) TFA/Phenol/Thioanisole/EDT/Me₂S/H₂O/NH₄I

### General Methods.

The Apelin (62-77) sequence was synthesized by standard automated SPPS on Fmoc-L-Phe-Wang resin (276 mg, 0.37 mmol/g) using an Applied Biosystem ABI 433A synthesizer (Appelar, France). The elongation was carried out by coupling of a 10-fold excess of Fmoc-L-amino acid derivatives, using 2-(1H-benzotriazol-1-yl)-1,1,3,3-tetramethyluronium hexafluorophosphate (HBTU), 1-hydroxybenzotriazole (HOBt), and diisopropylethylamine (Hünig's base) (DIPEA) as coupling reagents in N,N-dimethylformamide (DMF) as solvent. After each coupling step, Fmoc deprotection was performed by treatment with piperidine followed by UV at 301 nm. Lys(61) was introduced manually by coupling a 5-fold excess of either Fmoc-L-Lys(Boc)-OH or Boc-L-Lys(Fmoc)-OH, using HBTU, HOBt, and DIPEA as coupling reagents in DMF as solvent. Analytical reverse-phase high performance liquid chromatography (RP-HPLC) separations were performed on C18 Ascentis Express (2.7 µm, 4.6 mm × 75 mm) using a linear gradient (5% to 100% of solvent B in solvent A in 7.5 min, flow rate of 1.6 mL·min⁻¹, detection at 220 nm; solvent A: water/0.1% TFA; solvent B: acetonitrile/0.1% TFA). Semi-preparative reverse phase high performance liquid chromatography (RP-HPLC) separations were performed on a Waters XBridge RP-C18 column (5 µm, 19 × 100 mm) using a linear gradient (solvent B in solvent A; solvent A: water/0.1% TFA; solvent B: acetonitrile/0.1% TFA; flow rate of 20 mL·min⁻¹; detection at 220 nm). Purified compounds eluted as single and symmetrical peaks (thereby confirming a purity of ≥95%) at the retention times (*t*_{R}) given below. High resolution mass spectra (HRMS) were acquired on a Bruker MicroTof mass spectrometer, using electrospray ionization (ESI) and a time-of-flight analyzer (TOF).

### General protocols for the synthesis of alkyl- and perfluoroalkylpeptides.

Fmoc-L-Lys(Boc)-Ap(62-77)-Wang resin (1) or Boc-L-Lys(Fmoc)-FR(Pbf)R(Pbf)QR(Pbf)PR(Pbf)LS(tBu)H(Trt)K(Boc)GPMPF-Wang resin (2) (1 equiv) was swollen in DMF, and the excess solvent removed by filtration. A solution of piperidine in DMF (20% v/v-1 mL) was added, and the mixture was shaken at room temperature for 15 min. The solution was drained, and the operation was repeated for 15 min. The solution was drained, and the resin was washed with DMF and CH₂Cl₂. In a separate vial, DIPEA (5 equiv) was added to a solution of hexadecanoic acid or 4,4,5,5,6,6,7,7,8,8,9,9,10,10,11,11-heptadecafluoroundecanoic acid (2 equiv), HBTU (2 equiv), and HOBt (2 equiv) in DMF (1 mL). The mixture was stirred at room temperature for 1 min and was added to the resin. The mixture was shaken at room temperature for 90 min. The solution was drained, and the procedure was repeated for 90 min. The solution was drained and the resin was washed with DMF, CH₂Cl₂, and diethyl ether then dried *in vacuo.* The dried resin was treated with TFA/Phenol/Thioanisole/1,2-Ethanedithiol/Me₂S/water/NH₄I, 81/5/5/2.5/2/3/1.5 (reagent H, 2 mL), and the mixture was shaken at room temperature for 3 h. The solution was collected and the beads washed with TFA. The solution was evaporated *in vacuo* and the crude product purified by semipreparative RP-HPLC. Lyophilization afforded the expected product.

### Synthesis of JFM V-0196A.

Fmoc-L-Lys(Boc)-FR(Pbf)R(Pbf)QR(Pbf)PR(Pbf)LS(tBu)H(Trt)K(Boc)GPMPF-Wang resin (15 µmol), hexadecanoic acid (7.7 mg, 30 µmol), HBTU (11.3 mg, 30 µmol), HOBt (4.6 mg, 30 µmol), and DIPEA (13.1 µL, 75 µmol) were reacted according to the general procedure, affording the title compound (7.5 mg, 16%) as a white solid. *t*_{R} = 4.50 min. (>98% purity [220 nm]); HRMS (ESI) calcd for C₁₁₂H₁₉₁N₃₄O₂₁S ([M + 5H]⁵⁺) 476.09287; found, 476.09308.

### Synthesis of JFM V-0196B.

Fmoc-L-Lys(Boc)-FR(Pbf)R(Pbf)QR(Pbf)PR(Pbf)LS(tBu)H(Trt)K(Boc)GPMPF-Wang resin (15 µmol), 4,4,5,5,6,6,7,7,8,8,9,9,10,10,11,11-heptadecafluoroundecanoic acid (14.8 mg, 30 µmol), HBTU (11.3 mg, 30 µmol), HOBt (4.6 mg, 30 µmol), and DIPEA (13.1 µL, 75 µmol) were reacted according to the general procedure, affording the title compound (20.6 mg, 49%) as a white solid. *t*_{R} = 4.07 min. (>98% purity [220 nm]); HRMS (ESI) calcd for C₁₀₇H₁₆₄F₁₇N₃₄O₂₁S ([M + 5H]⁵⁺) 523.24519; found, 523.24507.

### Synthesis of JFM V-0220A.

Boc-L-Lys(Fmoc)-FR(Pbf)R(Pbf)QR(Pbf)PR(Pbf)LS(tBu)H(Trt)K(Boc)GPMPF-Wang resin (10 µmol), hexadecanoic acid (5.1 mg, 20 µmol), HBTU (7.5 mg, 20 µmol), HOBt (3.1 mg, 20 µmol), and DIPEA (8.7 µL, 50 µmol) were reacted according to the general procedure, affording the title compound (15.8 mg, 25%) as a white solid. *t*_{R} = 4.50 min. (>98% purity [220 nm]); HRMS (ESI) calcd for C₁₁₂H₁₉₁N₃₄O₂₁S ([M + 5H]⁵⁺) 476.09287; found, 476.09096.

### Synthesis of JFM V-0220B.

Boc-L-Lys(Fmoc)-FR(Pbf)R(Pbf)QR(Pbf)PR(Pbf)LS(tBu)H(Trt)K(Boc)GPMPF-Wang resin (10 µmol), 4,4,5,5,6,6,7,7,8,8,9,9,10,10,11,11-heptadecafluoroundecanoic acid (9.8 mg, 20 µmol), HBTU (7.5 mg, 20 µmol), HOBt (3.1 mg, 20 µmol), and DIPEA (8.7 µL, 50 µmol) were reacted according to the general procedure, affording the title compound (16.8 mg, 25%) as a white solid. *t*_{R} = 4.07 min. (>98% purity [220 nm]); HRMS (ESI) calcd for C₁₀₇H₁₆₄F₁₇N₃₄O₂₁S ([M + 5H]⁵⁺) 523.24519; found, 523.24349.

### II -Transfection and Establishment of Stable Cell Line

CHO-K1 (American Type Culture Collection; Rockville, MD, USA) cells were maintained in Ham's F12 medium supplemented with 10% fetal calf serum, 0.5 mM glutamine, 100 units/mL penicillin and 100 µg/mL streptomycin (all from Invitrogen, Carlsbad, CA, USA). Cells were transfected with plasmid coding for wild-type apelin receptor-EGFP, using Lipofectamine 2000 (Invitrogen), and stable cell line was established as previously described (42).

### III -Cell Membrane Preparations and Radioligand Binding Experiments

Crude membrane preparation from CHO stably expressing the wild-type rat apelin receptor-EGFP, were prepared as previously described (39). Membrane preparations (0.5-300 µg of total mass of membrane proteins/assay) were incubated for 60 min at 20° C with 2.10⁻¹⁰ M ¹²⁵I-pE13F (PerkinElmer Life Sciences) in binding buffer alone (50 mM Hepes, 5 mM MgCl₂, 1% BSA, pH 7.4) or in the presence of apelin or its analogs at various concentrations (10⁻¹⁴M to 10⁻⁴M). The reaction was stopped by adding ice-cold binding buffer and filtered through glass microfiber filters (Whatman GF/C filters). Radioactivity was counted in Wizard 1470 Wallac gamma counter (Perkin Elmer, Turku, Finland).

### IV - cAMP Assay

cAMP was quantified using the cAMP dynamic 2 assay kit (Cisbio Bioassays, Codolet, France) based on homogeneous time-resolved fluorescence (HTRF) technology. The stimulation was done in the stimulation buffer (HBSS, 5 mM Hepes, 0.1% BSA stabilizer, 1 mM IBMX, pH 7.4). Briefly, 2,000/well CHO cells stably expressing the rat apelin receptor-EGFP were added into 384-well plate and stimulated with 10⁻⁶ M forskolin (FSK) and increasing concentrations (10⁻¹⁴ to 10⁻⁴ M) of apelins or its analogs for 30 min at room temperature. Cells were then lyzed, and cAMP levels were determined following manufacturer instructions.

### V - Internalization Assays

The internalization assay was performed as described previously with CHO cells stably expressing the rat apelin receptor-EGFP (43). Briefly, cells were treated with 10⁻⁶ M apelins or its analogs, and internalization was triggered by incubating them at 37°C for 20 min. Cells were then mounted in Aquapolymount (Polysciences, Warrington, PA, USA) for confocal microscopic analysis (See (42) for details).

### VI - ERK1/2 phosphorylation assays

In order to compare the ability of K17F, pE13F, P92 and P26 to induce ERK1/2 phosphorylation, CHO cells stably expressing the wild-type apelin receptor-EGFP were treated with increasing concentrations of K17F, pE13F, P92 and P26 (from 10⁻¹¹ to 10⁻⁵ M) for 10 min. ERK1/2 phosphorylation was then monitored by Alphascreen technology.

### VII -Stability in mouse plasma.

Stability of K17F, pE13F, P26, P92, JFM V-0196A and JFM V-0196B was determined in mouse plasma at 37 °C. For each compound, the stock solution (100 µM in water) was diluted in plasma to a final incubation concentration of 5 µM. The incubation at 37 °C was stopped respectively at t₀ and 4 h by adding one volume of ice cold acetonitrile containing 0.1% trifluoroacetic acid. The sample was vortexed for 1 min and then centrifuged at 4 °C before LC-MS injection of the supernatant. Analyses were performed on a Kinetex RP-C₁₈ column (2.6 µm, 100 Å, 50 × 4.6 mm) using a linear gradient (solvent B in solvent A, solvent A: water/0.05% TFA; solvent B: acetonitrile; flow rate of 2 mL.min⁻¹; detection at 358 nm). The percentage of remaining test compound relative to t₀ was measured by monitoring the peak area of the chromatogram.

### VIII -Animals

Male Sprague Dawley rats (130-180 g BW), male adult Wistar rats (300-400 g), and male Swiss mice (18-20 g) were maintained under 12 h light-dark cycle with free access to food and water and were obtained from Charles River Laboratories (L'Arbresle, France). All animal experiments were carried out in accordance with current institutional guidelines for the care and use of experimental animals.

### IX - Microdissection of glomerular arterioles

The left kidney of male rats was prepared for microdissection of arterioles as previously described (44). Glomerular arterioles were isolated under stereomicroscopic observation. The afferent and muscular efferent arterioles were isolated with the glomerulus and identify according their morphology and localization in the inner renal cortex as previously described by *Helou et al.* (45).

### X - Measurement of glomerular arterioles diameter

For these experiments afferent and muscular efferent arterioles were microdissected attached to the gomeruli. Sequential photographies were recorded on a same arteriole with a digital camera (microscope LEICA DMRB fitted with a camera Nikon DXM1200) under three experimental conditions at one minute intervals: control, 10⁻⁹M Ang II and 10⁻⁹M Ang II + 5.10⁻⁷M P92. Arteriolar diameters were measured with Adobe Photoshop CS. Diameters were measured on a distance equal to about 100 µm upstream of the glomerulus and triplicate were performed for each arteriole. Calibration was made using a stage micrometer. The average diameter for each experimental condition was used for statistical analysis. Taking account of differences of arteriolar diameters between afferent and muscular efferent arterioles (45), the variations of arteriole diameters were expressed in percentage of controls.

### XI - Aortic Rings Preparation and Isometric Tension Recording

The experiments were performed in rat aortic rings as previously described (39). The rats were anaesthetized (pentobarbital sodium, 60 mg/kg by intraperitoneal route) and the thoracic aortas were carefully excised and placed in cold physiological saline solution (PSS) containing (mmol/L): 118.3 NaCl, 4.7 KCl, 2.5 CaCl₂, 1.2 MgSO₄, 1.2 KH₂PO₄, 25 NaHCO₃, 0.016 EDTA and 11.1 glucose. The aortas were cleaned of excess connective tissue and fat and cut into rings of approximately 3 - 4 mm in length. Special care was taken to avoid damaging the luminal surface of the endothelium. Aortic rings were suspended in 20 mL -jacketed organ baths filled with 20 mL of PSS continuously aerated with a mixture of 5% CO₂, 95% O₂, pH 7.4, at 37.4°C. One end of the aortic ring was connected to a tissue holder and the other to an isometric force transducer (EMKA Technologies, Paris, France). The rings were equilibrated for 120 min under a resting tension of 2 g. During equilibration period, the rings were washed every 30 min. Then, a first relaxation to acetylcholine (Ach, 10⁻⁴ mol/L) was implemented to check the integrity or the absence of the endothelium in rings precontracted with NA (3 × 10⁻⁸ mol/L). After rinsing with PSS to baseline tension, rings were equilibrated for 90 min. At the end of this equilibration period, cumulative concentration-response curves to K17F (10⁻¹² to 10⁻⁴ M), pE13F (10⁻¹² to 10⁻⁴ M), P26 (10⁻¹² to 10⁻⁴ M) or P92 (10⁻¹² to 10⁻⁴ M) were constructed after precontraction with NA (3 × 10⁻⁶ mol/L). Each concentration of the drug was added at the maximal effect of the precedent concentration. The concentration-response curves were continuously recorded on a PC by means of IOX v 2.4 (EMKA Technologies, Paris) for further analysis (Datanalyst v2.1, EMKA Technologies, Paris).

### XII - Blood pressure recording in anaesthetized Wistar rats

Wistar rats were anaesthesized with 100 mg/kg intraperitoneal (i.p.) inactin [5-ethyl-2-(1¢-methylpropyl)-2-thiobarbiturate] (RBI, IL, USA). Apelin fragments (K17F or P92) were dissolved in 0.2ml Krebs buffer (mM: NaCl 118.5, KCL 4.75, CaCl2 1.4, NaHCO3 24, MgSO4 1.19, KH2PO4 1.21, glucose 11). The resulting solution was administered to the rats via a catheter inserted into the right femoral vein, and was immediately followed by 0.2ml Krebs buffer alone to flush the venous catheter. An additional catheter was inserted into the right femoral artery, as previously described (47), for the monitoring of mean arterial blood pressure (MBP) as previously described (48). The arterial catheter was connected to a COBE CDX III pressure transducer (Phymep, Paris, France) linked to the Maclab system (Phymep, Paris, France). HR measurement was triggered by the blood pressure signal. BP was continuously recorded throughout the experiment. Each rat received an *i.v.* injection of apelin fragments, 15 min after the arterial catheter was connected to the pressure transducer.

The area under the curve of ΔMBP (AUC, area between baseline and mean BP) was calculated for each animal for the 15 minutes immediately following the injection. Mean AUC for each group were then calculated. Unpaired Student's t test was used to determine whether BP observed in response to substances administered (K17F or P92, i.v.) has statistically significant difference.

### XIII - Intracerebroventricular Injections in Mice and AVP Radioimmunoassay.

K17F (1 µg) and P92 (from 0.01 µg to 1 µg) were administrated by i.c.v. route in conscious mice with free access to water or deprived of water for 24 h as previously described (4). Animals were killed 1 min after the injection, and trunk blood (0.5-1 ml) was collected in chilled tubes containing 50 µl of 0.3 M EDTA pH 7.4. AVP concentrations were determined as previously described (4) from 0.2 ml of plasma by using a specific vasopressin-[Arg⁸] RIA kit (Peninsula Laboratories International Inc, San Carlo, USA).

### XIV - Isolated Perfused Rat Heart Preparation and Cardiac Contractility Recording

Animals were anaesthetized with pentobarbital sodium (50 mg/kg intraperitonally). Heparin (200 IU/kg) was administrated into the femoral vein. Hearts were excised quickly and arrested in ice-cold Krebs-Henseleit solution containing (mM): NaCl 118.5, KCl 4.75, MgSO₄ 1.19, KH₂PO₄ 1.2, NaHCO₃ 24, CaCl₂ 1.4 and glucose 11. Hearts were then mounted on a perfusion apparatus and retrograde perfusion was established via the ascending aorta at a constant flow rate of 6 ml/min with a peristaltic pump (Minipuls 3, model 172). The hearts were perfused with Krebs-Henseleit bicarbonate buffer which is bubbled with 95% O₂ / 5% CO₂ to keep pH 7.4 at 37°C. Temperature was continuously monitored by a thermoprobe inserted into the right atrium. Hearts beat spontaneously under the sinus rhythm. A domestic-food-wrap-made, fluid-filled, isovolumic balloon was introduced into the left ventricle through the left atrial appendage and inflated to give a preload of 8 to 10mmHg. Left ventricular pressure was recorded continuously on a computer through a data-acquisition system (Chart V5, Powerlab 16/30, ADInstruments, UK). The maximal rate of rise of left ventricular pressure (dP/dtmax) and heart rate were derived from left ventricular pressure. After 20-minutes equilibration period, the hearts were treated by drugs added to the perfusate with an infusion pump (Harvard Apparatus Pump 11) at rate of 100 µl/min for 30 minutes.

### XV- Data and statistical analysis

Data from the binding and cAMP experiments were analyzed with GraphPad Prism (GraphPad Software, La Jolla, CA, USA). Statistical comparisons were performed with Student's unpaired t-test or one-way analysis of variance (ANOVA) with Dunnet's post-test. Statistical differences for calcium measurement were assessed using Student's unpaired t-test or one-way analysis of variance (ANOVA) on weighted means followed by Fisher's test.

Values for aorta isometric tension recording are given as means ± sem. One-way ANOVA (comparison of Eₘₐₓ and pD₂) or ANOVA for repeated measures followed by a Fisher's protected least significance (comparison of concentration-response curves) was used to assess the significance of the results. P<0.05 was considered as significant.

### EXAMPLE: 2 Results

### 1 - Affinity of K17F, pE13F and apelin analogs for the rat apelin receptor-EGFP stably expressed in CHO cells

In order to protect pE13F from enzymatic degradation *in vivo,* we replacing each amino acid of pE13F with its D-isomer or with synthetic amino acids. We first determine which amino acid of pE13F could be replaced without affecting binding of the modified peptide to the apelin receptor. *Ki* values from D-scanning experiments for apelin receptor were 0.6 ± 0.1 nM for pE13F and 37.5 ± 11.3 nM, 40.2 ± 9.0 nM, 3.4 ± 0,4 nM, 23.3 ± 4.7 nM, 4.1 ± 2.0 nM, 12.2 ± 4.6 nM, 4.3 ± 1.8 nM, 8.6 ± 2.7 nM for pE13F(D-Arg²), pE13F(D-Arg⁴), pE13F(D-Leu⁵), pE13F(D-Ser⁶), pE13F(D-His⁷), pE13F(D-Lys⁸), pE13F(D-Ala⁹) and pE13(D-Phe¹³) respectively. We also obtained *Ki* values for apelin receptor of 1.4 ± 0.7 nM for Ac-R12F, 2.6 ± 2.3 nM for pE13F(Aib⁷), 0.8 ± 0.2 nM for pE13F(Nle¹¹) and 0.06 ± 0.02 nM for pE13(4Br-Phe¹³). The combination in pE13F of the deletion of pGlu and the addition of N-acetyl Arg², D-Leu⁵, Aib⁷, D-Ala⁹, Nle¹¹ and 4Br-Phe¹³ provided the compound P26 which exhibited a *Ki* value of 2.1 ± 0.4 nM (Table 2)

Table 1 showed the *Ki* values for the combination of the substitutions in K17F. The acetylation of Lys¹ together with the substitutions D-Leu⁹, Aib¹¹, D-Ala¹³, Nle¹⁵ and 4Br-Phe¹⁷ raised the compound P96 with an affinity of 0.11 ± 0.14 nM. The addition of the substitution in position 5, D-Gln⁵ still improves the affinity of the compound P95 (0.03 nM) as compared to K17F by a factor 10. Finally, the compound P92 in which we introduced, in addition to all the changes performed in P95, a D-Arg in position 3, exhibited an affinity of 0.21 ± 0.13 nM similar to that of K17F.

Finally, the *Ki* values for K17F and the analogs P26 and P92 were 0.3 ± 0.1 nM, 2.1 ± 0.4 nM, 0.2 ± 0.06 nM respectively (Table 2).

**Table 1. Pharmacological Characterization of K17F analogs**

| | **Amino acid sequences** | **[¹²⁵I] pE13F binding IC₅₀** (nM) | **cAMP production IC₅₀ (nM)** |
|---|---|---|---|
| **K17F** | **Lys-Phe-Arg-Arg-Gln-Arg-Pro-Arg-Leu-Ser-His-Lys-Gly-Pro-Met-Pro-Phe** | 0.29 ± 0.24 | 0.30 ± 0.10 |
| **P96** | N-Acetyl-**Lys-Phe-Arg-Arg-Gln-Arg-Pro-Arg**-D-Leu-Ser-Aib-**Lys**-D-Ala-**Pro**-Nle-**Pro**-(4-Br)Phe | 0.11 ± 0.14 | 0.45 ± 0.07 |
| **P95** | N-Acetyl-**Lys-Phe-Arg-Arg**-D-Gln-**Arg-Pro-Arg**-D-Leu-**Ser-**Aib-**Lys**-D-Ala-**Pro**-Nle-**Pro**-(4-Br)Phe | 0.03 ± 0.02 | 0.34 ± 0.36 |
| **P92** | N-Acetyl-**Lys-Phe**-D-Arg-**Arg**-D-Gln-**Arg-Pro-Arg**-D-Leu-Ser-Aib-**Lys**-D-Ala-**Pro**-Nle-**Pro**-(4-Br)Phe | 0.21 ± 0.13 | 0.62 ± 0.77 |

**Table 2: Development of metabolically stable apelin analogs**

| | **Amino acid sequences** | [¹²⁵I] **pE13F binding i (nM)** | **cAMP production IC₅₀ (nM)** | **Internalization EC₅₀ (nM)** | **ERK1/2 phospha on EC₅₀** |
|---|---|---|---|---|---|
| **pE13F** | **pGlu-Arg-Pro-Arg-Leu-Ser-His-Lys-Gly-Pro-Met-Pro-Phe** | 0.56 ± 0.07 | 1.68 ± 0.47 | 1.7 ± 1.2 | 12.0 ± |
| **P26** | N-Acetyl-**Arg-Pro-Arg**-DLeu-**Ser**-Aib-**Lys**-DAla-**Pro**-Nle-**Pro**(4-Br)Phe | 2.11 ± 0.40 | 2.22 ± 1.00 | 2.1 ± 1.1 | 72.1 ± |
| **K17F** | **Lys-Phe-Arg-Arg-Gln-Arg-Pro-Arg-Leu-Ser-His-Lys-Gly-Pro-Met-Pro-Phe** | 0.26 ± 0.12 | 0.30 ± 0.20 | 0.26 ± 0.09 | 4.08 ± |
| **P92** | N-Acetyl-**Lys-Phe**-DArg-**Arg**-DGln-**Arg-Pro-Arg**-DLeu-**Ser-**Aib-Lys-DAla-Pro-Nle-Pro-(4Br)Phe | 0.20 ± 0.06 | 0.56 ± 0.32 | 0.38 ± 0.11 | 3.42 ± |
| **JFM V-0196A** | CH₃(CH₂)₁₄C(O)-**Lys-Phe-Arg-Arg-Gln-Arg-Pro-Arg-Leu-Ser-His-Lys-Gly-Pro-Met-Pro-Phe** | 0.76 ± 0.13 | 3.9 ± 1.5 | | |
| **JFM V-0196B** | CF₃(CF₂)₇(CH₂)₂C(O)**-Lys-Phe-Arg-Arg-Gln-Arg-Pro-Arg-Leu-Ser-His-Lys-Gly-Pro-Met-Pro-Phe** | 0.21 ± 0.27 | 3.1 ± 1.43 | | |

### 2 - Effects on inhibition of forskolin-induced cAMP production in CHO cells stably expressing the rat apelin receptor-EGFP

Incubation of CHO cells stably expressing the rat apelin receptor-EGFP with 10⁻⁶ M forskolin in presence of increasing concentrations of K17F, pE13F and their analogs (10⁻¹⁴ to 10⁻⁶ M) resulted in a concentration-dependent inhibition of forskolin-induced cAMP production with IC₅₀ values of 1.0 ± 0.2 nM and 0.3 ± 0.2 nM for pE13F and K17F respectively and IC50 values of 927 ± 312 nM, 419 ± 58 nM, 25 ± 9.6 nM, 350 ± 180 nM, 317 ± 143 nM, 76 ± 10 nM, 43 ± 12 nM, 109 ± 27 nM for pE13F(D-Arg²), pE13F(D-Arg⁴), pE13F(D-Leu⁵), pE13F(D-Ser⁶), pE13F(D-His⁷), pE13F(D-Lys⁸), pE13F(D-Ala⁹) and pE13(D-Phe¹³) respectively. We also obtained an IC₅₀ value of 2.4 ± 1.4 nM for Ac-R12F, 1.2 ± 0.6 nM for pE13F (Aib⁷), 1.0 ± 0.5 nM for pE13F (Nle¹¹) and 0.1 ± 0.05 nM for pE13(4Br-Phe¹³). The K17F analogs P92, P95, P96 for their part, exhibited inhibitory potencies similar to that of K17F (Table 1). Finally, IC₅₀ values for the analogs P26 and P92 were 4.4 ± 0.9 nM and 0.2 ± 0.06 nM respectively (Table 2).

### 3 - Effects on the Internalization of the rat apelin receptor-EGFP stably expressed in CHO cells

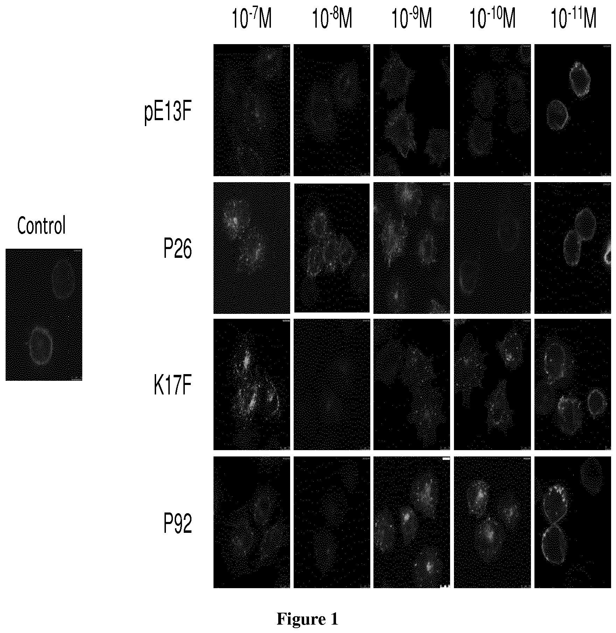

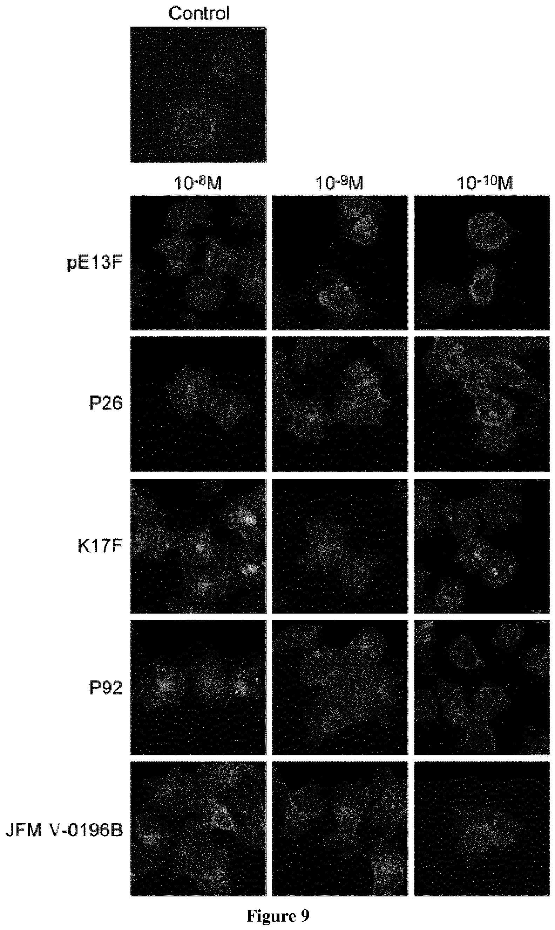

We also investigated the ability of pE13F or K17F and their analogs to induce internalization of the rat apelin receptor-EGFP in CHO cells stably expressing this receptor. Because recombinant apelin receptor was tagged at its C-terminal part with EGFP, we could visualize apelin receptor internalization by following the redistribution of the fluorescence from the plasma membrane compartment to small cytoplasmic fluorescent vesicles. Incubation of apelin receptor-EGFP stably transfected CHO cells with increasing concentrations of pE13F or K17F for 20 min, resulted in a progressive and marked endocytosis of the apelin receptor as shown by the disappearance of fluorescence at the plasma membrane and the appearance of numerous intracellular fluorescent vesicles, with EC50 values of 1.7 and 0.26 nM respectively (Table 3, Figure 1). In contrast, incubation of CHO cells stably expressing the rat apelin-receptor-EGFP with 1 µM pE13F(D-Arg²), pE13F(D-Arg⁴) and pE13F (D-Lys₈) did not induce apelin receptor internalization. Indeed, CHO cells displayed an intense apelin receptor-EGFP fluorescence at the level of the plasma membrane without intracellular fluorescent vesicles. In contrast, the analogs AcR12F, pE13F(Aib⁷), pE13F(D-Lys⁸), pE13F(D-Ala⁹), pE13F(Nle¹¹), pE13(4Br-Phe¹³) are potent inducers of apelin receptor internalization. Similarly, P26, P92 and JFM V-0196B were able to induce ApelinR internalization with EC₅₀ values of 2.11 ± 1.14, 0.38 ± 0.11 and 0.41 ± 0.16 nM, respectively (Tables 2, 7, Figures 1, 9). The quantification of the internalization induced by the analogs P26 and P92 compared to pE13F and K17F showed an order of efficiency: K17F=P92 > pE13F= P26.

### 4 - Effects of K17F, pE13F, P92 and P26 on ERKl/2 phosphorylation in CHO cells stably expressing the rat apelin receptor-EGFP.

Dose-response curves of ERK1/2 phosphorylation in response to K17F, pE13F, P92 and P26 showed a similar maximal effect for K17F and peptide 92 with equivalent EC₅₀ values of 4.08 ± 1.17 nM and 3.42 ± 2.41 nM, respectively, whereas EC₅₀ values for pE13F and peptide 26 are 3 and 18 times higher (EC₅₀ = 12.0 ± 2.79 10⁻⁹ M and 71.3 ± 19.1 10⁻⁹ M, respectively) than K17F (Table 2). Dose-response curves of ERK1/2 phosphorylation with the various compounds showed similar maximal effects with EC50 values of 4.08 ± 1.17 nM, 3.42 ± 2.41 nM and 0.89 ± 0.61 nM for K17F, P92 and JFM V-0196B, respectively (Table 7). In contrast, pE13F and P26 were weaker promoters of ERK1/2 phosphorylation with corresponding EC50 values of 12.01 ± 2.79 nM and 72.10 ± 19.60 nM, respectively (Table 7). Thus, the rank order of efficiency for the following compounds was JFM V-0196B > P92 = K17F > pE13F >>P26.

### 5 - Affinity of alkyl and perfluoroalkylpeptides derived from K17F for the rat apelin receptor-EGFP stably expressed in CHO cells and their inhibitory potency on forskolin -induced cAMP production

The affinity of the alkyl K17F-analogs for the apelin receptor was lower than that of the perfluoroalkylpeptides (Table 3).

**Table 3: Pharmacological caracterisation of alkyl and perfluoroalkylpeptides derived from K17F**

| **Composé** | **Peptide** | **KinM ± SEM** (15.11.2013) | **IC50 cAMP inhibition (nm)** |
|---|---|---|---|
| K17F | H-Lys-Phe-Arg-Arg-Gln-Arg-Pro-Arg-Leu-Ser-His-Lys-Gly-Pro-Met-Pro-Phe-OH | 0.049±0.008 (n = 7) | 0.09 ± 0.01 (n = 6) |
| JFM V-0196A | CH₃(CH₂)₁₄C(O)-Lys-Phe-Arg-Arg-Gln-Arg-Pro-Arg-Leu-Ser-His-Lys-Gly-Pro-Met-Pro-Phe-OH | 0.76 ± 0.13 (n = 4) | 3.9 ± 1.5 (n = 4) |
| JFM V-0196B | CF₃(CF₂)₇(CH₂)₂C(O)-Lys-Phe-Arg-Arg-Gln-Arg-Pro-Arg-Leu-Ser-His-Lys-Gly-Pro-Met-Pro-Phe-OH | 0.21 ± 0.027 (n = 4) | 3.1 ± 1.43 (n = 5) |
| JFM V-0220A | 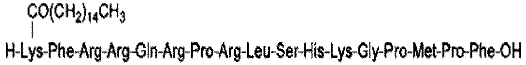 | 1.43 ± 0.74 (n =3) | 2 ± 0.75 (n = 3) |
| JFM V-0220B | 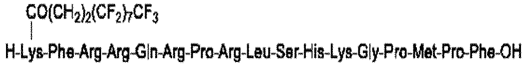 | 0.16 ± 0.010 (n = 3) | 1 ± 0.4 (n = 3) |
| **JFM V-0210/1** | 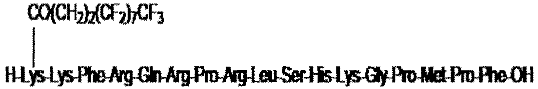 | **0.18 ± 0.023 (n = 3)** | **3.8 ± 1 (n = 5)** |
| **JFM Y 0210/2** | 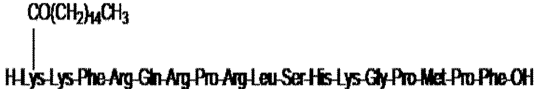 | **1.33 ± 0.15 (n = 3)** | **1.4 ± 0.5 (n = 3)** |

The best compounds are JFM V-0196B and JFM V-0220B with an affinity of 0.2 nM. The fact to add at the N-terminal part of K17F a Lysine residue with a perfluoroalkyl chain (compound JFM V-0210/1) does not affect the affinity as compare to K17F labelled with a perfluoroalkyl on the epsilon of its own Lysine. The inhibitory potency of these compounds on forskolin-induced cAMP production is in the nanomolar range. In conclusion, the fact to add an alkyl or a perfluoroalkyl group at the N-terminal part of K17F does not drastically modify its affinity (between a factor of 4 to 15) or its capacity to inhibit forskolin-induced cAMP production (between a factor of 15 to 40) (Table 3).

### 6 - Plasma half-life of, pE13F, P26, K17F, P92 and the compounds JFM V-0196A and JFM V-0196B

**Stability in mouse plasma.** Stability of K17F, pE13F, P26, P92, JFM V-0196A and JFM V-0196B was determined in mouse plasma at 37 °C (Table 4). Whereas the half-life values in plasma of K17F and pE13F are 4.6 and 7.2 min respectively, the respective derivative of K17F and pE13F: P92 and P26 display an increase plasma stability with half-life values of 24 and 86 min. Moreover, the alkyl or perfluoroalkyl derivates of K17F exhibit a higher plasma stability since, even after 4h of incubation no degradation was observed.

**Table 4. Mouse plasma stability of peptides JFM V-0196A and JFM V-0196B**

| **Compound** | **Half-life mouse plasma stability** |
|---|---|
| pE13F | 7.2 min |
| P26 | 86 min |
| K17F | 4.6 min |
| P92 | 24 min |
| JFM V-0196A | 100% of starting peptide after 4 h |
| M V-JFM V-0196B | 100% of starting ptide arfter 4 |
| | 100% of starting peptide after 4 h |

### 7 - Vasorelaxant effects of K17F, pE13F, P92 and P26

### on rat aortic rings precontracted by noradrenaline (NA)

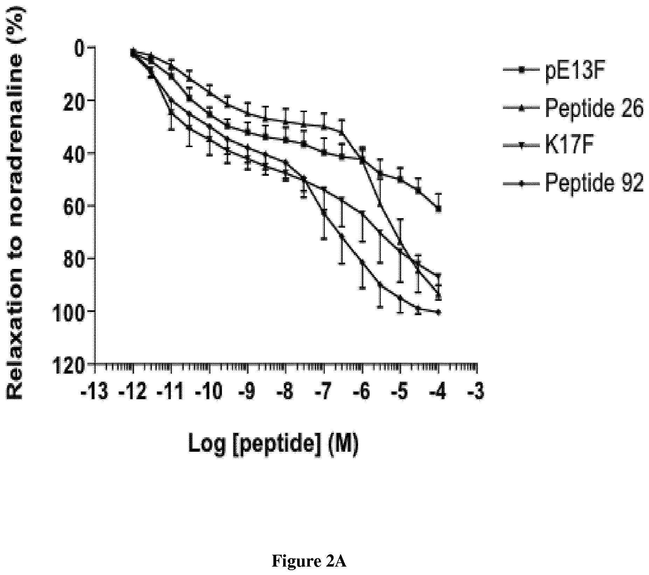

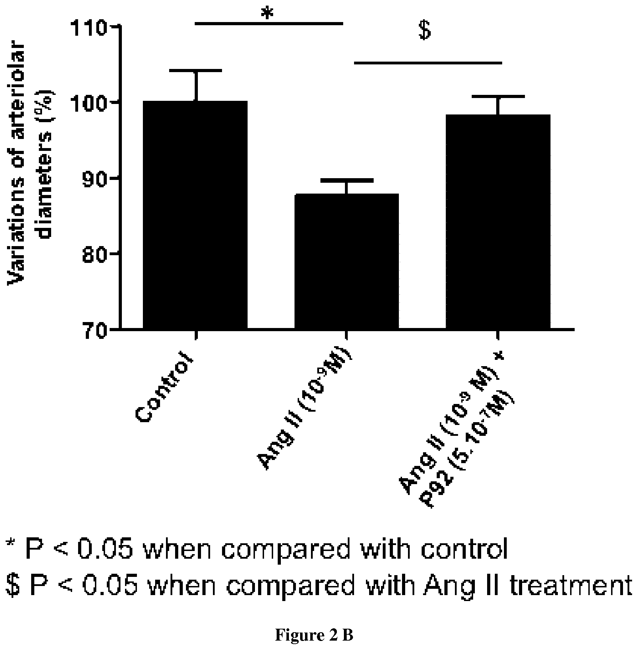

In rat aortic rings precontracted with 3×10⁻⁶ M NA, K17F, pE13F, P92 and P26 induced concentration-dependent relaxation (Figure 2). The potency (pD₂) of P26 (6.27 ± 0.42) was significantly (P<0.05) lower than that of K17F (8.30 ± 0.44), pE13F (8.00 ± 0.59) and P92 (7.68 ± 0.57). In contrast, the maximal effect (62 ± 6 %) induced by pE13F was significantly less than the maximal relaxant effects induced by K17F (87 ± 8 %, P<0.01), P26 (93 ± 3 %, P<0.001) and P92 (100 ± 1 %, P<0.001). (Figure 2A). In contrast, at a maximal concentration of 100 µM, the vasorelaxing effects induced by pE13F (62 ± 6 %) and JFM V-0196B (60 ± 3 %,) were significantly lower than that induced by K17F at the same concentration (93 ± 3 %, P<0.01) (Fig. 10A), showing a lower efficacy of these two compounds as compared to K17F.

### on rat glomerular arterioles precontracted by angiotensin II (Ang II)

To evaluate the effects of these compounds on the vascular reactivity of rat glomerular arterioles, diameters were measured 1) in basal conditions, 2) after adding Ang II and 3) in the presence of Ang II and P92. Results are presented on the Figure 2B and showed that 1nM Ang II significantly reduced the arteriolar diameter compared with values measured under baseline conditions (100.0 ± 4.1 vs 87.6 ± 1.9 %, n=5, P < 0.05, respectively). Addition of 500 nM P92 to preconstricted arterioles by 1 nM Ang II increased the arteriolar diameter from 87.6 ± 1.9 to 98.1 ± 2.5 % (n=5, p < 0.05). These results indicated that P92 as K17K was able to induce a vasodilatation of glomerular arterioles previously preconstricted by Ang II.

Moreover, application of 500 nM JFM V-196B to precontracted arterioles by 1 nM Ang II increased the arteriolar diameter from 12.97 ± 0.50 to 13.88 ± 0.45 µm, (n=5, p < 0.05). The vasorelaxant effects of K17F and P92 were blocked in the presence of 20 □M L-NAME, a NO synthase inhibitor, in contrast to that of JFM V-196B which was not significantly changed (Fig. 10B).

### 8- Effects of intravenous injection of K17F or P92 or JFM V - 0196B on arterial blood pressure in anaesthetized normotensive rats

### A) Effects of P92 on arterial blood pressure

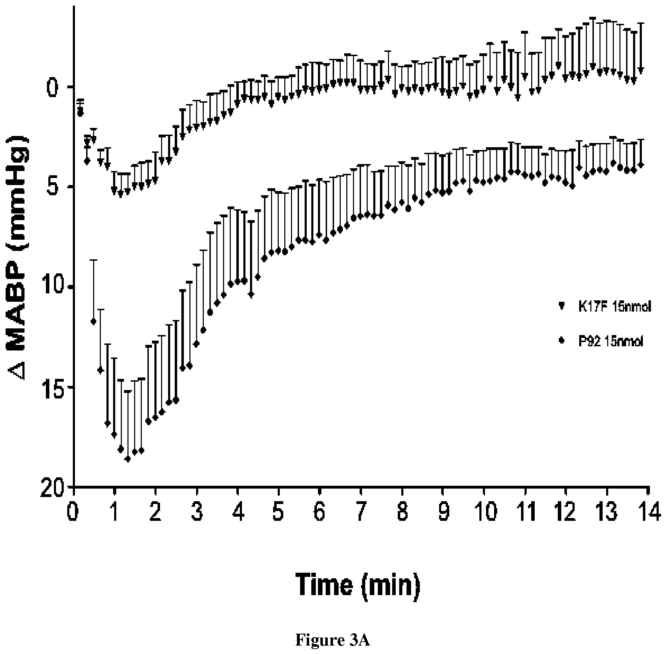

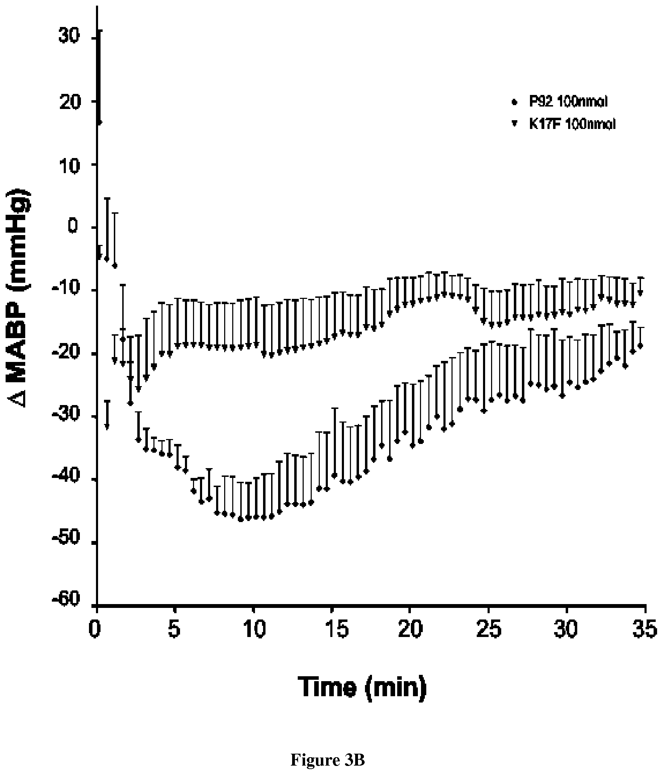

Basal MBP was 100.3 ± 1.2 mmHg in normotensive Wistar rats (300g) anaesthesized with inactin (dose 100 mg/kg). The intravenous injection of K17F (15 nmol/rat = 50 nmol/ kg) decreased mean arterial blood pressure (MABP) by 7.8 mmHg. The hypotensive response was maximal 1.2 min after injection and was only transient, probably reflecting the rapid degradation of the peptide in the bloodstream. A return to baseline was observed 4.2 min after injection. At a dose of 15 nmol/rat, P92 was much more effective than K17F at reducing BP (-20.8 mmHg in 1min, P<0.05 vs Wistar rats receiving 15nmol K17F), a return to baseline 10.8 min (P<0.05 vs. Wistar rats receiving 15 nmol K17F) after injection (Figure 3A). The intravenous injection of K17F (100 nmol/rat = 333 nmol/kg = 0.3 µg/kg) decreased MABP by 30.5 mmHg and a return to a plateau value of-12 mmHg observed around 22 min. For the P92 at the same dose, we observed a decrease in MABP of 55.4 mmHg with a return to a plateau value of - 20 mmHg observed around 35 min (Figure 3B).

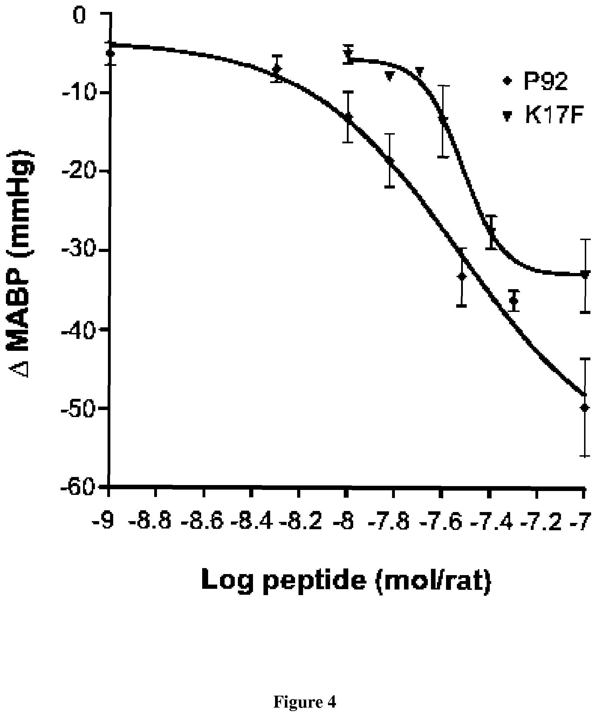

In Wistar rats, i.v. injection of P92 or K17F (5-100 nmol/rat) dose-dependently decreased MABP with an ED50 of 29.5 and 30 nmol respectively (Figure 4). The calculation of the AUC of BP response confirmed the more important hypotensive response in Wistar rats injected *i*.*v*. with P92, compared to rats with K17F (AUC after 100 nmol P92 *vs.* 100 nmol K17F: -34303 ± 4003 mmHg.s vs -13712 ± 5271 mmHg.s, P< 0.05).

JFM V-0196B, i.v. injected in increasing doses (from 5 to 15 nmol/rat correspond to 16.6 to 50 nmol/kg) in anaesthesized Wistar rats, dose-dependently decreased BP with a maximal decrease of - 51.4 ± 6.1 mmHg observed at 10 min for a dose of 15 nmol versus 5.4 ± 1 mmHg for K17F at the same dose. A slight decrease in BP (between 6 and 10 mmHg) was still observed at 108 min after the injection (not shown). The decrease in BP and the duration of the hypotensive effect at 15 nmol are respectively 9 and 27 fold higher than those of K17F at the same dose.

### B) Effects of JFM V - 0196B on arterial blood pressure

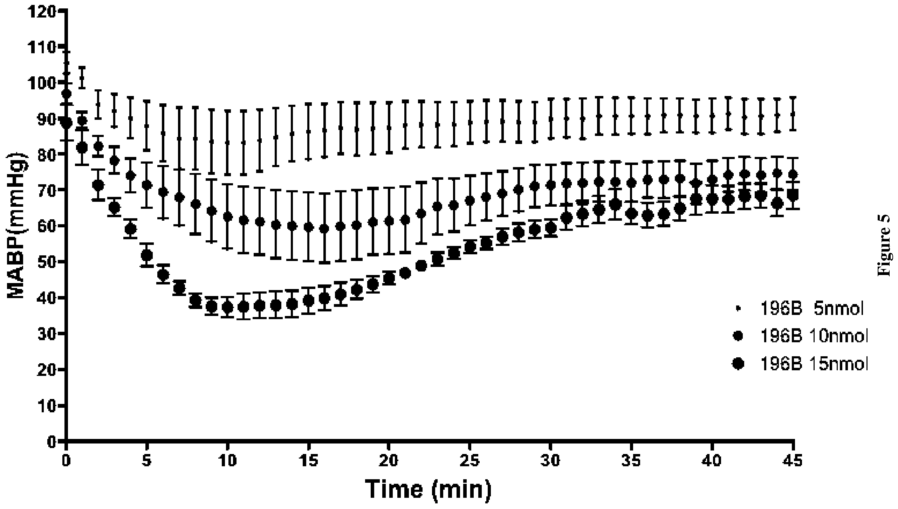

In a second series of experiments, we have measured the effects of the compound JFM V - 0196B on BP in anaesthesized Wistar normotensive rats (Figure 5). JFM V - 0196B, *i.v.* injected in increasing doses (from 5 to 15 nmol/rat) dose-dependently decreased BP with a maximal decrease of 50 mmHg for a dose of 15 nmol versus 7.8 mmHg for K17F. A return to a plateau value of - 25 mmHg was observed for 10 or 15 nmol P92 around 33 min. In contrast, in Wistar rats receiving 15 nmol K17F, a return to baseline was observed after 10.8 min.

**Table 5 Comparison of the maximal effects of 15 nmol K17F, P92, and JFM V-0196B, i.v. injected in anaesthesized normotensive rats**

| | **Maximal decrease in BP (mmHg)** | **Time for maximal BP decrease (min)** | **Time to return to a plateau value (min)** | |
|---|---|---|---|---|
| **K17F** | **- 7.8** | **1.2** | | **4.2 (*baseline*)** |
| **P92** | **- 20.8** | **1.6** | | **10.8 *(-12 mmHg)*** |
| **JFM V-0196B** | **- 52** | **10** | | **33 *(-21 mmHg)*** |

The maximal hypotensive response and the duration of the hypotensive effect of the compound P92, after i.v. injection in anaesthesized normotensive rats, are 3 fold higher than that of K17F (Table 5). For the compound JFM V - 0196B, the maximal hypotensive response and the duration of the hypotensive effect are respectively 6.7 and 8 fold higher than those of K17F knowing that after 30 min, at the plateau value, there is still a BP decrease of 21 mmHg. Additional experiments are needed to define the time of return to baseline after P92 and JFM V - 0196B injection (Table 5).

### 9 - Effects of intracerebroventricular (icv) injection of K17F P92 or JFMV-196B in alert euhydrated or dehydrated mice on AVP release in the blood circulation

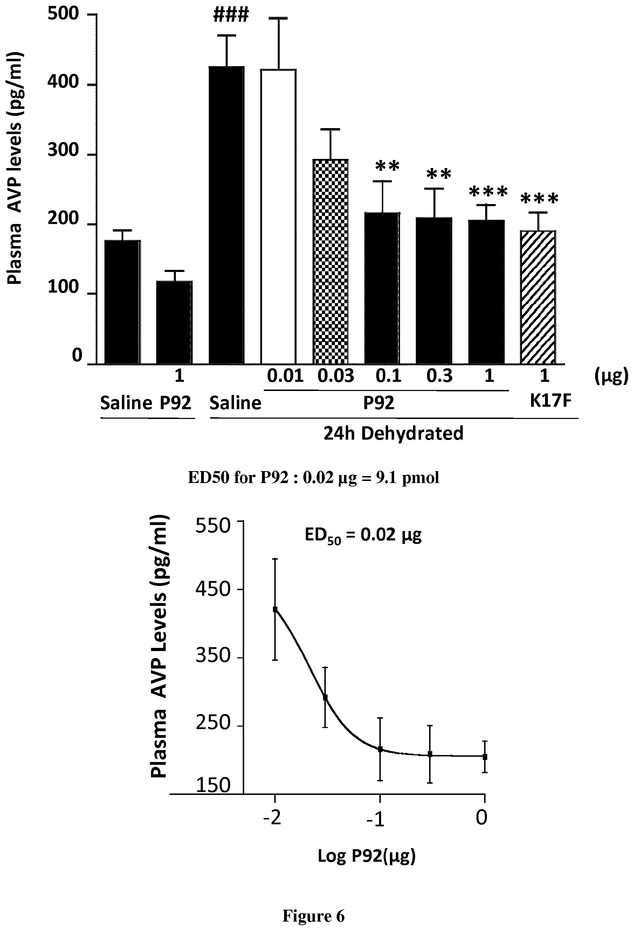

Water deprivation of mice for 24 h significantly increases plasma AVP levels into two sets of experiments (425 ± 46 pg/ml, n=18 versus control mice 175 ±17 pg/ml, n=20; P< 0.001, Fig. 6A&B and 644 ± 60 pg/ml, n=14 versus control mice 232 ± 54 pg/ml, n=8; P< 0.001, Fig. 6A&B). As previously described {Iturrioz, 2010 #32}, i.c.v. injection of K17F in water-deprived mice at the dose of 1 µg (468 pmol) significantly decreased plasma AVP levels (190 ± 27 pg/ml, n=7) compared with water-deprived mice injected with saline (425 ± 46 pg/ml) (P<0.001) (Fig. 6).

I.c.v. injection of P92 in increasing doses (0.01 µg to 1 µg corresponding to 4.5 to 454 pmol) to water-deprived mice induced a dose-dependent decrease in plasma AVP levels. The ED50 for P92 (0.02 µg = 9.1 pmol; Fig. 6) was lower by a factor 6 compared to that of K17F (ED50 = 56 pmol f Iturrioz, 2010 #32}). The maximal decrease in AVP release induced by P92 observed for a dose of 0.1 µg (45 pmol/mouse) of P92 (- 85 %) was similar to that observed with 1 µg of K17F (468 pmol/mouse) K17F (- 94 %) (Fig. 11). I.c.v. injection of JFM V-0196B in increasing doses (0.001 µg to 0.03 µg corresponding to 0.29 to 8.79 pmol) to water-deprived mice induced a progressive decrease in plasma AVP levels (Fig. 11). The maximal decrease in AVP release induced by JFM V-0196B observed for a dose of 0.01 µg (2.93 pmol/mouse) of JFM V-0196B (- 75 %). The ED50 for JFM V-0196B (0.001 µg = 0.29 pmol; Fig. 11) was lower by a factor 193 than that of K17F (ED50 = 56 pmol). P92 and JFM V-0196B i.c.v injected alone in euhydrated mice at a supramaximal dose of 1 µg have no effect on plasma AVP levels.

### 10 - Effects of K17F or P92 on cardiac contractility in isolated perfused rat heart preparation

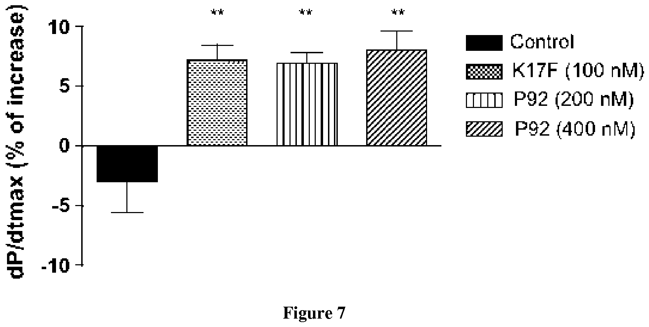

Recording of left ventricular pressure from isolated rat heart revealed that K17F (0.01 to 300 nM) dose-dependently increased developed pressure. The maximal effect was observed for a dose comprised between 100 and 300 nM. On the other hand P92 at the doses of 200 and 400 nM increase cardiac contractility with an amplitude similar to K17F (Figure 7). No chronotropic effect was observed for the two compounds tested.

**Table 6: Useful amino acid sequences for practicing the invention**

| **SEQ ID NO** | **amino acid sequence (in bold Isomer D or non natural amino-Acid)** | **Associated with compound** |
|---|---|---|
| **1** | KFRRQRPRLSHKGPMPF | *K17F, JFM V-0196A, JFM V-0196B, JFM V-220A, JFM V-220B, JFM V-210*/*1; JFMV-210*/*2* |
| **2** | KF**R**_{D}R**Q_{D}**RPR**L_{D}**S**Aib**K**A_{D}**PN**le**P(**4-Br**)**F** | **P92, FLUORO-P92, LIPO-P92** |
| **3** | KFRR**Q_{D}**RPR**L_{D}**S**Aib**K**A_{D}**P**Nle**P(**4-Br**)**F** | **P95** |
| **4** | KFRDRQRPR**L_{D}**S**Aib**K**A_{D}**P**Nle**P(**4-Br**)**F** | **P96** |
| **5** | pERPRL SHKGPMPF | **pE13F** |
| **6** | RPR**L_{D}**S**Aib**K**A_{D}**P**Nle**P(**4-Br**)**F** | **P26** |

**Table 7: Pharmacological characterization of metabolically stable apelin analogs**

| **Apelin analogs** | **Amino acid sequences** | | | | |
|---|---|---|---|---|---|
| **pE13F** | pGlu-Arg-Pro-Arg-Leu-Ser-His-Lys-Gly-Pro-Met-Pro-Phe-OH | | | | |
| **P26** | **N-Acetyl**-Arg-Pro-Arg-**DLeu**-Ser-**Aib**-Lys-**DAla**-Pro-**Nle-Pro-(4-Br)Phe**-OH | | | | |
| **K17F** | H-Lys-Phe-Arg-Arg-Gin-Arg-Pro-Arg-Leu-Ser-His-Lys-Gly-Pro-Met-Pro-Phe-OH | | | | |
| **P92** | N-Acetyl-Lys-Phe-DArg-Arg-DGln-Arg-Pro-Arg-DLeu-Ser-Aib-Lys-DAIa-Pro-Nle-Pro-(4Br)Phe-OH | | | | |
| **JFM V-0196B** | **CF₃(CF₂)₇(CH₂)₂C(O)**-Lys-Phe-Arg-Arg-Gln-Arg-Pro-Arg-Leu-Ser-His-Lys-Gly-Pro-Met-Pro-Phe-OH | | | | |

| **Apelin analogs** | **Binding affinity *K*i (nM)** | **Inhibition of FSK-induced cAMP production IC₅₀ (nM)** | **Internalization EC₅₀ (nM)** | **ERK1/2 phosphorylation EC₅₀ (nM)** | **Half-life in plasma (min)** |
|---|---|---|---|---|---|
| **pE13F** | 0.56 ± 0.07 | 1.68 ± 0.47 | 1.72 ± 1.23 | 1201 ± 2.79 | 7.2 |
| **P26** | 2.11 ± 0.40 | 2.22 ± 100 | 2.11 ± 1.14 | 72.10 ± 19.60 | 86 |
| **K17F** | 0.06 ± 0.01 | 0.30 ± 0.10 | 0.26 ± 0.09 | 4.08 ± 1.17 | 4.6 |
| **P92** | 0.09 ± 0.02 | 0.56 ± 0.32 | 0.38 ± 0.11 | 3.42 ± 2.41 | 24 |
| **JFM V-0196B** | 0.08 ± 0.01 | 3.13 ± 1.43 | 0.41 ± 0.16 | 0.89 ± 0.61 | > 240 |

Binding affinity values (Ki), inhibitory potency (IC50) of FSK-induced cAMP production, internalization potency (EC50) and ERK1/2 phosphorylation capacity of the peptides represent the mean ± S.E.M. from at least 3 independent experiments performed in duplicate or triplicate. Additional experiments were performed as compared to Table 2 for determining Ki values of K17F, P92 and JFM V-196B in parallel to those of fluoro P92 and Lipo P92.

### Example: 3 Binding tests of compounds FLUORO-P92 and LIPO-P92:

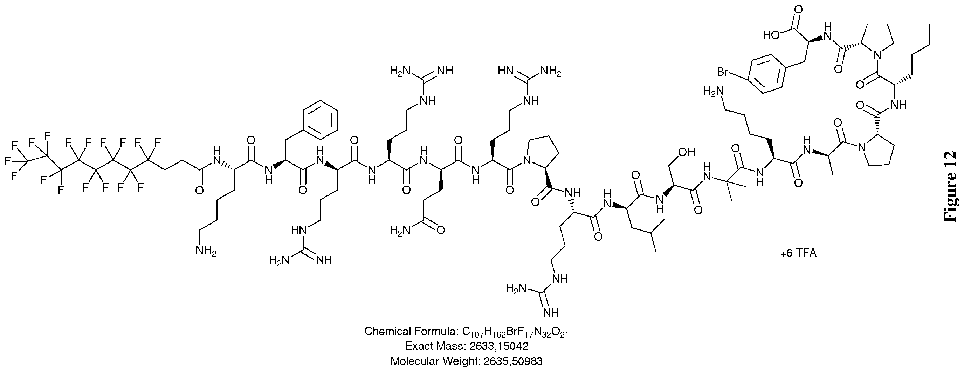

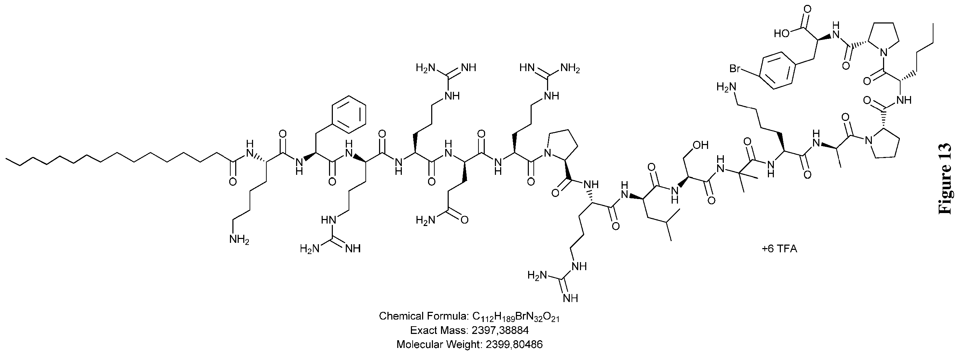

These compounds are synthesized as described in example 1 for other compounds of the present invention. The chemical structures of FLUORO-P92 and LIPO-P92 are disclosed in Figure 12 and Figure 13 respectively.

The affinity of compounds FLUORO-P92 and LIPO-P92 for the apelin receptor was checked on membrane preparation from CHO cells stably expressing the rat apelin receptor-EGFP. These compounds exhibit a high affinity in the subnanomolar range. The Ki value of FLUORO-P92 is 0.31 ± 0 .05 nM whereas that of LIPO-P92 is 0.25 ± 0 .02 nM is decreased only by a factor 2 to 3 as compared to P92 (Ki value 0.09 nM).

### REFERENCES:

Throughout this application, various references describe the state of the art to which this invention pertains.
1. De Mota, N., Z. Lenkei, and C. Llorens-Cortes, Cloning, pharmacological characterization and brain distribution of the rat apelin receptor. Neuroendocrinology 2000; 72(6): p. 400-7.
2. O'Dowd, B.F., M. Heiber, A. Chan, et al., A human gene that shows identity with the gene encoding the angiotensin receptor is located on chromosome 11. Gene 1993; 136(1-2): p. 355-60.
3. Tatemoto, K., M. Hosoya, Y. Habata, et al., Isolation and characterization of a novel endogenous peptide ligand for the human APJ receptor. Biochem Biophys Res Commun 1998; 251(2): p. 471-6.
4. De Mota, N., A. Reaux-Le Goazigo, S. El Messari, et al., Apelin, a potent diuretic neuropeptide counteracting vasopressin actions through inhibition of vasopressin neuron activity and vasopressin release. Proc Natl Acad Sci U S A 2004; 101(28): p. 10464-9.
5. Habata, Y., R. Fujii, M. Hosoya, et al., Apelin, the natural ligand of the orphan receptor APJ, is abundantly secreted in the colostrum. Biochim Biophys Acta 1999; 1452(1): p. 25-35.
6. Kawamata, Y., Y. Habata, S. Fukusumi, et al., Molecular properties of apelin: tissue distribution and receptor binding. Biochim Biophys Acta 2001; 1538(2-3): p. 162-71.
>C Lee, D.K., R. Cheng, T. Nguyen, et al., Characterization of apelin, the ligand for the APJ receptor. J Neurochem 2000; 74(1): p. 34-41.
8. Mesmin, C., F. Fenaille, F. Becher, J.C. Tabet, and E. Ezan, Identification and characterization of apelin peptides in bovine colostrum and milk by liquid chromatography-mass spectrometry. J Proteome Res 2011; 10(11): p. 5222-31.
9. El Messari, S., X. Iturrioz, C. Fassot, et al., Functional dissociation of apelin receptor signaling and endocytosis: implications for the effects of apelin on arterial blood pressure. J Neurochem 2004; 90(6): p. 1290-301.
10. Reaux, A., K. Gallatz, M. Palkovits, and C. Llorens-Cortes, Distribution of apelinsynthesizing neurons in the adult rat brain. Neuroscience 2002; 113(3): p. 653-62.
11. O'Carroll, A.M., T.L. Selby, M. Palkovits, and S.J. Lolait, Distribution of mRNA encoding B78/apj, the rat homologue of the human APJ receptor, and its endogenous ligand apelin in brain and peripheral tissues. Biochim Biophys Acta 2000; 1492(1): p. 72-80.
12. Reaux, A., N. De Mota, I. Skultetyova, et al., Physiological role of a novel neuropeptide, apelin, and its receptor in the rat brain. J Neurochem 2001; 77(4): p. 1085-96.
13. Medhurst, A.D., C.A. Jennings, M.J. Robbins, et al., Pharmacological and immunohistochemical characterization of the APJ receptor and its endogenous ligand apelin. J Neurochem 2003; 84(5): p. 1162-72.
14. Hus-Citharel, A., N. Bouby, A. Frugiere, et al., Effect of apelin on glomerular hemodynamic function in the rat kidney. Kidney Int 2008; 74(4): p. 486-94.
15. Hus-Citharel, A., L. Bodineau, A. Frugiere, et al., Apelin Counteracts Vasopressin-Induced Water Reabsorption via Cross Talk Between Apelin and Vasopressin Receptor Signaling Pathways in the Rat Collecting Duct. Endocrinology 2014; 155(11): p. 4483-93.
16. Reaux-Le Goazigo, A., A. Morinville, A. Burlet, C. Llorens-Cortes, and A. Beaudet, Dehydration-induced cross-regulation of apelin and vasopressin immunoreactivity levels in magnocellular hypothalamic neurons. Endocrinology 2004; 145(9): p. 4392-400.
17. Azizi, M., X. Iturrioz, A. Blanchard, et al., Reciprocal regulation of plasma apelin and vasopressin by osmotic stimuli. J Am Soc Nephrol 2008; 19(5): p. 1015-24.
18. Blanchard, A., O. Steichen, N. De Mota, et al., An abnormal apelin/vasopressin balance may contribute to water retention in patients with the syndrome of inappropriate antidiuretic hormone (SIADH) and heart failure. J Clin Endocrinol Metab 2013; 98(5): p. 2084-9.
19. Tatemoto, K., K. Takayama, M.X. Zou, et al., The novel peptide apelin lowers blood pressure via a nitric oxide-dependent mechanism. Regul Pept 2001; 99(2-3): p. 87-92.
20. Ishida, J., T. Hashimoto, Y. Hashimoto, et al., Regulatory roles for APJ, a seventransmembrane receptor related to angiotensin-type 1 receptor in blood pressure in vivo. J Biol Chem 2004; 279(25): p. 26274-9.
21. Ashley, E.A., J. Powers, M. Chen, et al., The endogenous peptide apelin potently improves cardiac contractility and reduces cardiac loading in vivo. Cardiovasc Res 2005; 65(1): p. 73-82.
22. Berry, M.F., T.J. Pirolli, V. Jayasankar, et al., Apelin has in vivo inotropic effects on normal and failing hearts. Circulation 2004; 110(11 Suppl 1): p. II187-93.
23. Foldes, G., F. Horkay, I. Szokodi, et al., Circulating and cardiac levels of apelin, the novel ligand of the orphan receptor APJ, in patients with heart failure. Biochem Biophys Res Commun 2003; 308(3): p. 480-5.
24. Iwanaga, Y., Y. Kihara, H. Takenaka, and T. Kita, Down-regulation of cardiac apelin system in hypertrophied and failing hearts: Possible role of angiotensin II-angiotensin type 1 receptor system. J Mol Cell Cardiol 2006; 41(5): p. 798-806.
25. Kuba, K., L. Zhang, Y. Imai, et al., Impaired heart contractility in Apelin gene-deficient mice associated with aging and pressure overload. Circ Res 2007; 101(4): p. e32-42.
26. Dickstein, K., A. Cohen-Solal, G. Filippatos, et al., ESC Guidelines for the diagnosis and treatment of acute and chronic heart failure 2008: the Task Force for the Diagnosis and Treatment of Acute and Chronic Heart Failure 2008 of the European Society of Cardiology. Developed in collaboration with the Heart Failure Association of the ESC (HFA) and endorsed by the European Society of Intensive Care Medicine (ESICM). Eur Heart J 2008; 29(19): p. 2388-442.
27. Lloyd-Jones, D., R.J. Adams, T.M. Brown, et al., Executive summary: heart disease and stroke statistics--2010 update: a report from the American Heart Association. Circulation 2010; 121(7): p. 948-54.
28. Roger, V.L., A.S. Go, D.M. Lloyd-Jones, et al., Heart disease and stroke statistics-2011 update: a report from the American Heart Association. Circulation 2011; 123(4): p. e18-e209.
29. Loehr, L.R., W.D. Rosamond, P.P. Chang, A.R. Folsom, and L.E. Chambless, Heart failure incidence and survival (from the Atherosclerosis Risk in Communities study). Am J Cardiol 2008; 101(7): p. 1016-22.
30. Effects of enalapril on mortality in severe congestive heart failure. Results of the Cooperative North Scandinavian Enalapril Survival Study (CONSENSUS). The CONSENSUS Trial Study Group. N Engl J Med 1987; 316(23): p. 1429-35.
31. Effect of enalapril on survival in patients with reduced left ventricular ejection fractions and congestive heart failure. The SOLVD Investigators. N Engl J Med 1991; 325(5): p. 293-302.
32. The Cardiac Insufficiency Bisoprolol Study II (CIBIS-II): a randomised trial. Lancet 1999; 353(9146): p. 9-13.
33. Effect of metoprolol CR/XI, in chronic heart failure: Metoprolol CR/XI, Randomised Intervention Trial in Congestive Heart Failure (MERIT-HF). Lancet 1999; 353(9169): p. 2001-7.
34. Consensus recommendations for the management of chronic heart failure. On behalf of the membership of the advisory council to improve outcomes nationwide in heart failure. Am J Cardiol 1999; 83(2A): p. 1A-38A.
35. Packer, M., M.R. Bristow, J.N. Cohn, et al., The effect of carvedilol on morbidity and mortality in patients with chronic heart failure. U.S. Carvedilol Heart Failure Study Group. N Engl J Med 1996; 334(21): p. 1349-55.
36. Levy, D., S. Kenchaiah, M.G. Larson, et al., Long-term trends in the incidence of and survival with heart failure. N Engl J Med 2002; 347(18): p. 1397-402.
37. Roger, V.L., S.A. Weston, M.M. Redfield, et al., Trends in heart failure incidence and survival in a community-based population. Jama 2004; 292(3): p. 344-50.
38. Devic, E., K. Rizzoti, S. Bodin, B. Knibiehler, and Y. Audigier, Amino acid sequence and embryonic expression of msr/apj, the mouse homolog of Xenopus X-msr and human APJ. Mech Dev 1999; 84(1-2): p. 199-203.
39. Iturrioz, X., R. Alvear-Perez, N. De Mota, et al., Identification and pharmacological properties of E339-3D6, the first nonpeptidic apelin receptor agonist. Faseb J 2010; 24(5): p. 1506-17.
40. Karlin, S. and S.F. Altschul, Methods for assessing the statistical significance of molecular sequence features by using general scoring schemes. Proc Natl Acad Sci U S A 1990; 87(6): p. 2264-8.
41. Pitkin, S.L., J.J. Maguire, T.I. Bonner, and A.P. Davenport, International Union of Basic and Clinical Pharmacology. LXXIV. Apelin receptor nomenclature, distribution, pharmacology, and function. Pharmacol Rev 2010; 62(3): p. 331-42.
42. Lenkei, Z., A. Beaudet, N. Chartrel, et al., A highly sensitive quantitative cytosensor technique for the identification of receptor ligands in tissue extracts. J Histochem Cytochem 2000; 48(11): p. 1553-64.
43. Iturrioz, X., R. Gerbier, V. Leroux, et al., By interacting with the C-terminal Phe of apelin, Phe255 and Trp259 in helix VI of the apelin receptor are critical for internalization. J Biol Chem 2010; 285(42): p. 32627-37.
44. Le Bouffant, F., A. Hus-Citharel, and F. Morel, Metabolic CO2 production by isolated single pieces of rat distal nephron segments. Pflugers Arch 1984; 401(4): p. 346-53.
45. Helou, C.M. and J. Marchetti, Morphological heterogeneity of renal glomerular arterioles and distinct [Ca2+]i responses to ANG II. Am J Physiol 1997; 273(1 Pt 2): p. F84-96.
46. Gaudet, E., J. Blanc, and J.L. Elghozi, Role of angiotensin II and catecholamines in blood pressure variability responses to stress in SHR. Am J Physiol 1996; 270(6 Pt 2): p. R1265-72.
47. Reaux, A., M.C. Fournie-Zaluski, C. David, et al., Aminopeptidase A inhibitors as potential central antihypertensive agents. Proc Natl Acad Sci U S A 1999; 96(23): p. 13415-20.

## Claims

1. An apelin analogue having the peptide of the following formula (I):
Lysine-Phenylalanine-Xaa1-Arginine-Xaa2-Arginine-Proline-Arginine-Xaa3-Serine- Xaa4-Lysine-XaaS-Proline-Xaa6- Proline-Xaa7 (I), wherein :
- a fluorocarbon group , an acetyl group, or an acyl group - with the following structure: CH3-CyHx-C(O)-, where y = 7 to 30, x = 2y, is linked to said peptide, directly or through a spacer selected from the group consisting of Lysine and Arginine, either on the alpha-amino or the epsilon-amino group of the first lysine residue of the peptide of formula (I), and when the spacer is a Lysine, the fluorocarbon group or acetyl group or acyl group is directly linked either on the alpha-amino or the epsilon-amino group of said spacer,
and wherein
Xaa1 is arginine (**R**) or D-isomer arginine (**R**_{D}).
Xaa2 is glutamine (**Q**) or D-isomer glutamine (**Q**_{D})
Xaa3 is leucine (**L**) or D-isomer Leucine (**L**_{D}).
Xaa4 is histidine (**H**) or α-aminoisobutyric acid (Aib),
Xaa5 is alanine (**A**) or D-isomer alanine (**A**_{D}) or glycine (**G**).
Xaa6 is Methionine (**M**), or Norleucine (Nle).
Xaa7 is phenylalanine (**F**) or 4-Br phenylalanine (**F**),
and wherein said fluorocarbon group linked to said peptide has the following structure: CmFn-CyHx-(L)-, where m = 3 to 30, n <= 2m + 1, y = 0 to 15, x <= 2y, (m + y) = 3- 30 and (L) which is optional, is a functional group resulting from covalent attachment to the peptides, preferably a carbonyle -C(O)- to form an amide bond to a lysine of said peptide.

2. The apelin analogue of claim 1, which is a metabolically stable analogue.

3. The apelin analogue of claim 1 or 2 wherein Xaa1 is D-isomer arginine (**R**_{D}).

4. The apelin analogue according to any one of claims 1 to 3 wherein Xaa2 is D-isomer glutamine (**Q**_{D}).

5. The apelin analogue according to any one of claims 1 to 4 wherein Xaa3 is Leucine (**L**_{D}).

6. The apelin analogue according to any one of claims 1 to 5 wherein Xaa4 is α-aminoisobutyric acid (Aib).

7. The apelin analogue according to any one of claims 1 to 6 wherein Xaa5 is D-isomer alanine (**A**_{D}).

8. The apelin analogue according to any one of claims 1 to 7 wherein Xaa6 is Norleucine (Nle).

9. The apelin analogue according to any one of claims 1 to 8 wherein Xaa7 is 4-Br phenylalanine (**F**).

10. The apelin analogue according to any one of claims 1 to 9, which is :

Acetyl-Lys-Phe-(D-Arg)-Arg-(D-Gln)-Arg-Pro-Arg-(D-Leu)-Ser-Aib-Lys-
(D-Ala)-Pro-Nle-Pro-(4-Br)Phe.

11. The apelin analogue according to claim 1 or 2, wherein the peptide is:
a peptide with the amino acid sequence of SEQ ID NO:1 (KFRRQRPRLSHKGPMPF),
and wherein, in the peptide a fluorocarbon group or an acyl group RC(O)-,is directly linked at the NH2 terminal residue of said peptide or at the NH2ε of the first lysine residue, or at the εNH2 of the lysine residue of the linker L Lysine.

12. The apelin analogue according to any one of claims 1 to 11 for use as a drug.

13. The apelin analogue according to claims 1 to 11, for use in a method for the treatment of a disease mediated by the Apelin receptor selected from the group consisting of:
- Cardiovascular diseases: Heart failure, kidney diseases (e.g. renal failure, nephritis, etc.) hypertension, pulmonary hypertension, cirrhosis, arteriosclerosis, pulmonary emphysema, pulmonary oedema; stroke, brain ischemia, myocardial impairment in sepsis;
- the syndrome of inappropriate antidiuretic hormone (SIADH) including pathologies like neurogenic diabetes mellitus (e.g. diabetic complications such as diabetic nephropathy, diabetic neuropathy, diabetic retinopathy, etc.), septic choc, thirst troubles;
- sarcopenia;
- polycystic kidney disease;
- hyponatremia.

14. The apelin analogue for use according to claim 13, for use in a method for the treatment of cardiovascular diseases and/or SIADH

15. The apelin analogue for use according to claim 14 wherein the cardiovascular diseases are selected from the group consisting of heart failure, kidney failure, hypertension, pulmonary hypertension.

16. A pharmaceutical composition, comprising an apelin analogue according to any one of claims 1-11, and one or more pharmaceutically acceptable excipient.

17. A metabolically stable apelin analogue according to claims 1 to 11 or a pharmaceutical composition thereof according to claim 16 for use in the treatment or in the prevention of a disease, condition or disorder mediated by the apelin in a mammal in need thereof.

## Patentansprüche

1. Apelinanalog mit dem Peptid der folgenden Formel (I):
Lysin-Phenylalanin-Xaa1-Arginin-Xaa2-Arginin-Prolin-Arginin-Xaa3-Serin-Xaa4-Lysin-XaaS-Prolin-Xaa6- Prolin-Xaa7 (I), wobei:
eine Fluorkohlenstoffgruppe, eine Acetylgruppe oder eine Acylgruppe - mit der folgenden Struktur: CH3-CyHx-C(O)-, wo y = 7 bis 30, x = 2y, an das Peptid, direkt oder durch einen Abstandhalter, der ausgewählt ist aus der Gruppe bestehend aus Lysin und Arginin, entweder auf der Alpha-Amino- oder der Epsilon-Aminogruppe des ersten Lysinrests des Peptids von Formel (I) gebunden ist, und wenn der Abstandhalter ein Lysin ist, die Fluorkohlenstoffgruppe oder Acetylgruppe oder Acylgruppe direkt entweder auf der Alpha-Amino- oder der Epsilon-Aminogruppe des Abstandhalters gebunden ist, und wobei
Xaa1 Arginin (**R**) oder D-Isomer-Arginin (**R**_{D}) ist,
Xaa2 Glutamin (**Q**) oder D-Isomer-Glutamin (**Q**_{D}) ist,
Xaa3 Leucin (**L**) oder D-Isomer-Leucin (**L**_{D}) ist,
Xaa4 Histidin (**H**) oder α-Aminoisobuttersäure (Aib) ist,
Xaa5 Alanin (**A**) oder D-Isomer-Alanin (**A**_{D}) oder Glycin (**G**) ist,
Xaa6 Methionin (**M**) oder Norleucin (Nle) ist,
Xaa7 Phenylalanin (**F**) oder 4-Br Phenylalanin (**F**) ist,
und wobei die Fluorkohlenstoffgruppe, die an das Peptid gebunden ist, die folgende Struktur aufweist: CmFn-CyHx-(L)-, wo m = 3 bis 30, n <= 2m + 1, y = 0 bis 15, x <= 2y, (m + y) = 3-30 und (L), das optional ist, eine funktionelle Gruppe ist, die aus kovalenter Anhaftung an die Peptide resultiert, vorzugsweise ein Carbonyl -C(O)-, um eine Amidbindung an ein Lysin des Peptids zu bilden.

2. Apelinanalog nach Anspruch 1, das ein metabolisch stabiles Analog ist.

3. Apelinanalog nach Anspruch 1 oder 2, wobei Xaa1 D-Isomer-Arginin (**R**_{D}) ist.

4. Apelinanalog nach einem der Ansprüche 1 bis 3, wobei Xaa2 D-Isomer-Glutamin (**Q**_{D}) ist.

5. Apelinanalog nach einem der Ansprüche 1 bis 4, wobei Xaa3 Leucin (**L**_{D}) ist.

6. Apelinanalog nach einem der Ansprüche 1 bis 5, wobei Xaa4 α-Aminoisobuttersäure (Aib) ist.

7. Apelinanalog nach einem der Ansprüche 1 bis 6, wobei Xaa5 D-Isomer-Alanin (**A**_{D}) ist.

8. Apelinanalog nach einem der Ansprüche 1 bis 7, wobei Xaa6 Norleucin (Nle) ist.

9. Apelinanalog nach einem der Ansprüche 1 bis 8, wobei Xaa7 4-Br Phenylalanin (**F**) ist.

10. Apelinanalog nach einem der Ansprüche 1 bis 9, das wie folgt ist:

Acetyl-Lys-Phe-(D-Arg)-Arg-(D-Gln)-Arg-Pro-Arg-(D-Leu)-Ser-Aib-Lys-(D-Ala)-Pro-
Nle-Pro-(4-Br)Phe.

11. Apelinanalog nach Anspruch 1 oder 2, wobei das Peptid ist:
ein Peptid mit der Aminosäuresequenz von SEQ ID NR:1 (KFRRQRPRLSHKGPMPF),
und wobei in dem Peptid eine Fluorkohlenstoffgruppe oder eine Acylgruppe RC(O)-direkt an den NH2-endständigen Rest des Peptids oder an das NH2ε des ersten Lysinrests oder an das εNH2 des Lysinrests des Linkers L-Lysin gebunden ist.

12. Apelinanalog nach einem der Ansprüche 1 bis 11 zur Verwendung als ein Arzneimittel.

13. Apelinanalog nach Ansprüchen 1 bis 11 zur Verwendung in einem Verfahren für die Behandlung einer Erkrankung, die durch den Apelinrezeptor vermittelt wird, ausgewählt aus der Gruppe bestehend aus:
- kardiovaskulären Erkrankungen: Herzinsuffizienz, Nierenerkrankungen (z.B. Nierenversagen, Nephritis usw.) Bluthochdruck, Lungenhochdruck Zirrhose, Arteriosklerose, Lungenemphysem, Lungenödem; Schlaganfall, Gehirnischämie, myokardiale Schädigung bei Sepsis;
- dem Syndrom einer inadäquaten Sekretion des antidiuretischen Hormons (SIADH), beinhaltend Pathologien wie neurogener Diabetes mellitus (z.B. diabetische Komplikationen wie diabetische Nephropathie, diabetische Neuropathie, diabetische Retinopathie usw.), septischer Schock, Durstbeschwerden;
- Sarkopenie;
- polyzystische Nierenerkrankung;
- Hyponaträmie.

14. Apelinanalog zur Verwendung nach Anspruch 13 zur Verwendung in einem Verfahren für die Behandlung von kardiovaskulären Erkrankungen und/oder SIADH.

15. Apelinanalog zur Verwendung nach Anspruch 14, wobei die kardiovaskulären Erkrankungen ausgewählt sind aus der Gruppe bestehend aus Herzinsuffizienz, Nierenversagen, Bluthochdruck, Lungenhochdruck.

16. Pharmazeutische Zusammensetzung, umfassend ein Apelinanalog nach einem der Ansprüche 1-11 und einen oder mehrere pharmazeutisch annehmbare Trägerstoffe.

17. Metabolisch stabiles Apelinanalog nach Ansprüchen 1 bis 11 oder pharmazeutische Zusammensetzung davon nach Anspruch 16 zur Verwendung in der Behandlung oder in der Prävention einer Erkrankung, eines Zustands oder einer Störung, die bzw. der durch das Apelin vermittelt wird, in einem Säugetier mit Bedarf daran.

## Revendications

1. Analogue d'apéline présentant le peptide de formule (I) suivante :
Lysine-Phénylalanine-Xaa1-Arginine-Xaa2-Arginine-Proline-Arginine-Xaa3-Sérine-Xaa4-Lysine-Xaa5-Proline-Xaa6-Proline-Xaa7 (1), dans lequel :
un groupe fluorocarbone, un groupe acétyle, ou un groupe acyle - ayant la structure suivante : CH3-CyHx-C(O)-, où y = 7 à 30, x = 2y, est lié audit peptide, directement ou par l'intermédiaire d'un espaceur sélectionné dans le groupe consistant en une lysine et une arginine, sur le groupe amino en alpha ou amino en epsilon du premier résidu de lysine du peptide de formule (I), et quand l'espaceur est une lysine, le groupe fluorocarbone ou le groupe acétyle ou le groupe acyle est directement lié au groupe amino en alpha ou amino en epsilon dudit espaceur, et dans lequel
Xaa1 est une arginine (**R**) ou l'isomère D de l'arginine (**R**_{D}).
Xaa2 est une glutamine (**Q**) ou l'isomère D de la glutamine (**Q**_{D})
Xaa3 est une leucine (**L**) ou l'isomère D de la leucine (**L**_{D}).
Xaa4 est une histidine (**H**) ou l'acide α-aminoisobutyrique (Aib),
Xaa5 est une alanine (**A**) ou l'isomère D de l'alanine (**A**_{D}) ou une glycine (**G**).
Xaa6 est une méthionine (**M**), ou une norleucine (Nle).
Xaa7 est une phénylalanine (**F**) ou une 4-Br-phénylalanine (**F**),
et dans lequel ledit groupe fluorocarbone lié audit peptide présente la structure suivante : CmFn-CyHx-(L)-, où m = 3 à 30, n <= 2m + 1, y = 0 à 15, x <= 2y, (m + y) = 3-30 et (L) qui est facultatif, est un groupe fonctionnel résultant d'une fixation covalente aux peptides, de préférence un carbonyle -C(O)- pour former une liaison amide avec une lysine dudit peptide.

2. Analogue d'apéline selon la revendication 1, qui est un analogue métaboliquement stable.

3. Analogue d'apéline selon la revendication 1 ou 2 dans lequel Xaa1 est l'isomère D de l'arginine (**R**_{D}).

4. Analogue d'apéline selon l'une quelconque des revendications 1 à 3 dans lequel Xaa2 est l'isomère D de la glutamine (**Q**_{D}).

5. Analogue d'apéline selon l'une quelconque des revendications 1 à 4 dans lequel Xaa3 est une Leucine (**L**_{D}).

6. Analogue d'apéline selon l'une quelconque des revendications 1 à 5 dans lequel Xaa4 est un acide α-aminoisobutyrique (Aib).

7. Analogue d'apéline selon l'une quelconque des revendications 1 à 6 dans lequel Xaa5 est l'isomère D de l'alanine (**A**_{D}).

8. Analogue d'apéline selon l'une quelconque des revendications 1 à 7 dans lequel Xaa6 est la norleucine (Nle).

9. Analogue d'apéline selon l'une quelconque des revendications 1 à 8 dans lequel Xaa7 est une 4-Br-phénylalanine (**F**).

10. Analogue d'apéline selon l'une quelconque des revendications 1 à 9, qui est :

Acétyl-Lys-Phe-(D-Arg)-Arg-(D-Gln)-Arg-Pro-Arg-(D-Leu)-Ser-Aib-Lys-(D-Ala)-Pro-
Nle-Pro-(4-Br)Phe.

11. Analogue d'apéline selon la revendication 1 ou 2, dans lequel le peptide est :
un peptide ayant la séquence d'acides aminés de SEQ ID NO : 1 (KFRRQRPRLSHKGPMPF),
et dans lequel, dans le peptide, un groupe fluorocarbone ou un groupe acyle RC(O)-, est directement lié au résidu terminal NH2 dudit peptide ou au NH2ε du premier résidu de lysine, ou au εNH2 du résidu de lysine du lieur L Lysine.

12. Analogue d'apéline selon l'une quelconque des revendications 1 à 11 pour une utilisation comme médicament.

13. Analogue d'apéline selon les revendications 1 à 11, pour une utilisation dans un procédé de traitement d'une maladie médiée par le récepteur de l'apéline sélectionné dans le groupe consistant en :
- les maladies cardiovasculaires : insuffisance cardiaque, maladies rénales (par exemple, insuffisance rénale, néphrite, etc.), hypertension, hypertension pulmonaire, cirrhose, artériosclérose, emphysème pulmonaire, oedème pulmonaire; accident vasculaire cérébral, ischémie cérébrale, insuffisance myocardique dans la septicémie ;
- le syndrome de l'hormone antidiurétique inappropriée (SIADH) incluant les pathologies comme le diabète sucré neurogène (par exemple, les complications diabétiques telles que la néphropathie diabétique, la neuropathie diabétique, la rétinopathie diabétique, etc.), le choc septique, les troubles de la soif ;
- la sarcopénie ;
- la polykystose rénale ;
- l'hyponatrémie.

14. Analogue d'apéline pour une utilisation selon la revendication 13, pour une utilisation dans un procédé de traitement des maladies cardiovasculaires et/ou du SIADH.

15. Analogue d'apéline pour une utilisation selon la revendication 14, dans lequel les maladies cardiovasculaires sont sélectionnées dans le groupe consistant en l'insuffisance cardiaque, l'insuffisance rénale, l'hypertension, l'hypertension pulmonaire.

16. Composition pharmaceutique, comprenant un analogue d'apéline selon l'une quelconque des revendications 1-11, et un ou plusieurs excipients pharmaceutiquement acceptables.

17. Analogue d'apéline métaboliquement stable selon les revendications 1 à 11 ou une composition pharmaceutique de celui-ci selon la revendication 16 pour une utilisation dans le traitement ou dans la prévention d'une maladie, d'une affection ou d'un trouble médié par l'apéline chez un mammifère en ayant besoin.
